# EUROPEAN PATENT APPLICATION

(11) **EP 4 501 360 A1**
(43) Date of publication of application: **05.02.2025**
(21) Application number: 23780186.5
(22) Date of filing: 24.03.2023
(51) Int. Cl.: A61K 47/20, A61K 9/10, A61K 9/14, A61K 31/7088, A61K 47/14, A61K 47/24, A61K 47/28, A61K 48/00, A61P 37/04

(54) **LIPID NANOPARTICLES FOR DELIVERING NUCLEIC ACID TO SPLENIC TISSUE, AND METHOD FOR DELIVERING NUCLEIC ACID TO SPLENIC TISSUE USING SAME**

(30) Priority: 28.03.2022 JP 2022051918
(71) Applicant: NOF Corporation, Shibuya-ku Tokyo 150-6019 (JP); Tohoku University, Sendai-shi, Miyagi 980-8577 (JP); National University Corporation Chiba University, Chiba-shi, Chiba 263-8522 (JP)
(72) Inventor: NAKAI, Yuta, Kawasaki-shi, Kanagawa 210-0865 (JP); TANGE, Kota, Kawasaki-shi, Kanagawa 210-0865 (JP); SAKURAI, Yu, Sendai-shi, Miyagi 980-8577 (JP); AKITA, Hidetaka, Sendai-shi, Miyagi 980-8577 (JP); TANAKA, Hiroki, Chiba-shi, Chiba 260-8675 (JP); HORI, Mizuho, Chiba-shi, Chiba 260-8675 (JP); GOMI, Masaki, Chiba-shi, Chiba 260-8675 (JP)
(74) Representative: Weickmann & Weickmann PartmbB
(86) International application number: PCT/JP2023/011850
(87) International publication number: WO 2023/190176

(57) **Abstract**

The present invention provides lipid nanoparticles for delivering nucleic acid to spleen tissue that can improve the efficiency of nucleic acid delivery to spleen tissue cells, and a method for delivering nucleic acid to spleen tissue by using same. A lipid nanoparticle for use in delivering a nucleic acid to a spleen tissue, including
(A) an ionic lipid represented by the formula (1),
(B) an anionic phospholipid or a compound represented by the formula (2),
(C) cholesterol, and
(D) a dimyristoylglycerol PEG represented by the formula:

CH₂(OR⁶)-CH(OR⁷)-CH₂(OR⁸)

(definitions of symbols in the formula are as described in the specification), and a method for delivering a nucleic acid to a spleen tissue by using same.

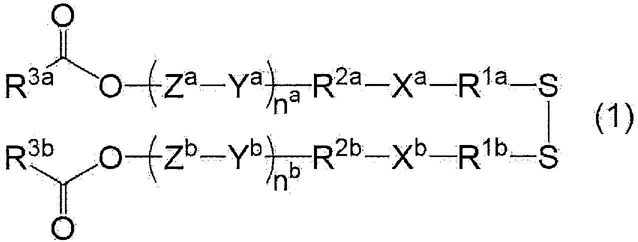

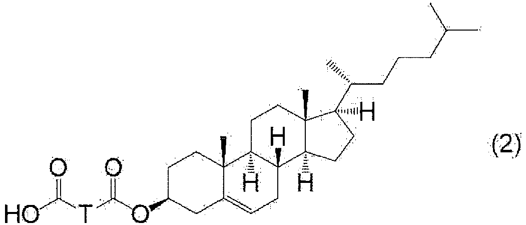

## Description

### [Technical Field]

The present invention relates to lipid nanoparticles for use in delivering nucleic acid to spleen tissues, and a method for delivering nucleic acid to spleen tissues by using same.

### [Background Art]

For practicalization of nucleic acid therapy using oligonucleic acids such as siRNA, and gene therapy using mRNA, pDNA, and the like, an effective and safe nucleic acid delivery carrier is demanded. Viral vectors are nucleic acid delivery carriers with high expression efficiency, but have safety problems in practical use. Therefore, the development of non-viral nucleic acid delivery carriers that can be used more safely is ongoing. Among them, lipid nanoparticles, which are carriers using ionic lipids, are currently the most commonly used non-viral nucleic acid delivery carriers.

Ionic lipids are broadly constituted of an amine moiety and a lipid moiety. For example, in ionic lipids, the amine moiety that protonates under acidic conditions and the polyanionic nucleic acid interact electrostatically to form lipid nanoparticles, which in turn promotes cellular uptake and delivers the nucleic acid into cells.

A known ionic lipid that is widely used in general is 1,2-dioleoyloxy-3-dimethylaminopropane (DODAP). It is known that by combining such known ionic lipid with phospholipid, cholesterol, and PEG lipid, lipid nanoparticles can be formed and nucleic acids can be delivered into cells (see, for example, Non Patent Literature 1).

For example, Patent Literature 1 describes an ionic lipid having a structure in which compounds consisting of one or two amine moieties and one lipid moiety are connected by a biodegradable disulfide bond. Patent Literature 1 shows that the ionic lipid improves pharmacokinetics such as blood stability, tumor targeting performance and the like. In addition, it has been shown that, by changing the structure around the amine moiety, pKa of the lipid membrane structure can be adjusted to a value favorable for endosomal escape within the cell, and that the cleavage of disulfide bonds within the cell can be used to dissociate nucleic acids from the lipid membrane structure. In fact, since the ionic lipid shows a higher nucleic acid delivery efficiency than the known ionic lipid DODAP, it has been revealed that the ionic lipid can improve intracellular dynamics, such as improved delivery efficiency of nucleic acids into the cytoplasm.

For example, Patent Literature 2 shows a lipid membrane structure that uses an ionic lipid that has an aromatic ring introduced near the lipid moiety in addition to a tertiary amine moiety and disulfide bond, thereby improving the ability to fuse with the endosomal membrane and further increasing the efficiency of nucleic acid delivery to the cytoplasm.

As described above, ionic lipids that improve intracellular dynamics by increasing endosomal escape efficiency and membrane fusion ability have been developed. On the other hand, for lipid nanoparticles made of ionic lipids to be more effective as nucleic acid delivery carriers in vivo, they need to have directivity to targeted organs and cells.

One of the PEG lipids widely used in lipid nanoparticles is dimyristoylglycerol PEG (DMG-PEG). It is known that when lipid nanoparticles using this are administered into the blood, the PEG lipid gradually dissociates from the lipid nanoparticles in the blood, and apolipoprotein E (ApoE) present in the blood adheres to the lipid nanoparticles, which increases accumulation in the liver which expresses the ApoE receptor (see, for example, Non Patent Literature 2).

An example of imparting directivity to organs other than the liver is the use of distearoylglycerol PEG (DSG-PEG), which has a hydrophobic group derived from stearic acid, as the PEG lipid, instead of DMG-PEG, which has a hydrophobic group derived from myristic acid, thereby enhancing accumulation in tumors (Non Patent Literature 3). Compared to DMG-PEG, DSG-PEG is less likely to dissociate from lipid nanoparticles in the blood, and therefore avoids adhesion of ApoE in the blood, suppresses accumulation in the liver, and exhibits high blood retention property, resulting in increased accumulation in tumor.

Another example of imparting directivity to organs other than the liver is the improvement of nucleic acid introduction efficiency to the spleen by controlling the particle size of lipid nanoparticles to 140 to 230 nm (see, for example, Patent Literature 3). It has been shown that lipid nanoparticles with a particle size of 200 nm avoid nucleic acid introduction into the liver and improve the efficiency of nucleic acid introduction into the spleen compared to lipid nanoparticles with a particle size of 100 nm.

As described above, the nucleic acid introduction efficiency can be increased by using lipid nanoparticles with improved intracellular dynamics, and nucleic acids can be efficiently delivered to target tissues such as liver, spleen and the like by changing the PEG lipid that is the component of lipid nanoparticles or adjusting the particle size of the lipid nanoparticles.

### [Citation List]

### [Patent Literature]

[Patent Literature 1]
   US Patent No. 9708628
[Patent Literature 2]
   WO 2019/188867
[Patent Literature 3]
   US 2020/0345641 A

### [Non Patent Literature]

[Non Patent Literature 1]
   Molecular Therapy, 25(7):1467-1475 (2017)
[Non Patent Literature 2]
   J. Control. Release, 235: 236-244 (2016)
[Non Patent Literature 3]
   J. Control. Release, 200: 97-105 (2015)
[Non Patent Literature 4]
   Biomater. Sci, 9: 1449-1463 (2021)

### [Summary of Invention]

### [Technical Problem]

Incidentally, the spleen is an organ that contains many immune cells and is therefore a potential target for nucleic acid vaccine applications. However, despite advances in this field, the efficiency of nucleic acid delivery to the spleen using conventional lipid nanoparticles is insufficient and there is room for improvement.

In addition, while the lipid nanoparticles described in Patent Literature 1 have been shown to have high efficiency of nucleic acid delivery to the liver, nucleic acid delivery to the spleen is not shown.

Non Patent Literature 4 and Patent Literature 3 each show the use of ionic lipids with different structural features as the lipid nanoparticles for delivery to the spleen. In these documents, however, different phospholipids are used as components of lipid nanoparticles, and the optimal combination with phospholipids varies depending on the structure of the ionic lipids.

As mentioned above, there are examples where the efficiency of nucleic acid introduction into spleen has been improved using lipid nanoparticles. However, because the appropriate phospholipid varies depending on the structure of the ionic lipid, the improvement is not fully satisfactory for practical use, and further improvements in nucleic acid delivery efficiency are required.

In view of the above-mentioned problems, the present invention aims to provide lipid nanoparticles for delivering nucleic acid to spleen tissue cells that can improve the efficiency of nucleic acid delivery to spleen tissue, and a method for delivering nucleic acid to spleen tissue by using same.

### [Solution to Problem]

As described above, the appropriate combination of phospholipid in lipid nanoparticles varies depending on the structure of ionic lipid and efficient nucleic acid delivery requires optimizing the combination of ionic lipid and phospholipid and the like.

The present inventors have conducted intensive studies in view of the above-mentioned problems and found that lipid nanoparticles produced using an ionic lipid that has a pKa suitable for endosomal escape and that is specifically decomposed in a reductive environment within a cell, and an anionic phospholipid or an anionic cholesterol represented by the formula (2), can efficiently introduce nucleic acids into spleen tissues. The present invention based on this finding is as follows.
[1] A lipid nanoparticle for use in delivering a nucleic acid to a spleen tissue, comprising
   (A) an ionic lipid represented by the formula (1): 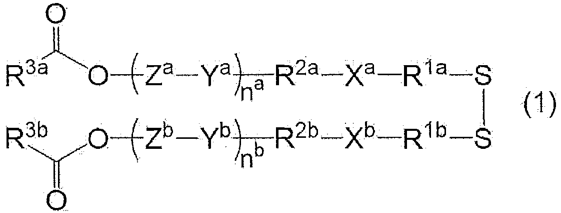 (in the formula (1),
      R^{1a} and R^{1b} are each independently an alkylene group having 1 - 6 carbon atoms,
      X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
      R^{2a} and R^{2b} are each independently an alkylene group or an oxydialkylene group each having not more than 8 carbon atoms,
      Y^{a} and Y^{b} are each independently an ester bond, an amide bond, a carbamate bond, an ether bond or a urea bond,
      Z^{a} and Z^{b} are each independently a divalent group derived from an aromatic compound having 3 - 16 carbon atoms and at least one aromatic ring, and optionally having a hetero atom,
      n^{a} and n^{b} are each independently 0 or 1, and
      R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

         R⁹-O-CO-(CH₂)a- (4)

         (in the formula (4),
         R⁹ is an aliphatic hydrocarbon group having 2 - 20 carbon atoms, and
         a is an integer of 2 - 10)),
   (B) an anionic phospholipid or a compound represented by the formula (2): 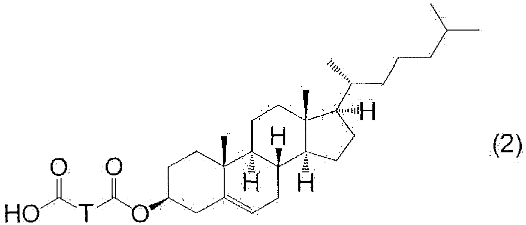 (in the formula (2),
      T is a divalent aliphatic hydrocarbon group having 1 - 8 carbon atoms),
   (C) cholesterol, and
   (D) a dimyristoylglycerol PEG represented by the formula (3):

      CH₂(OR⁶)-CH(OR⁷)-CH₂(OR⁸) (3)

      (in the formula (3),
      any two of R⁶, R⁷ and R⁸ are myristoyl groups, and the remaining one is an alkyl group having 1 - 6 carbon atoms connected via a polyethylene glycol chain with a number average molecular weight of 1,000 to 3,000).
[2] The lipid nanoparticle of [1], wherein the aforementioned anionic phospholipid is 1,2-diacyl-sn-glycero-3-phosphoglycerol or 1,2-diacyl-sn-glycero-3-phosphoserine.
[3] The lipid nanoparticle of [1] or [2], wherein the aforementioned anionic phospholipid is at least one selected from the group consisting of 1,2-dimyristoyl-sn-glycero-3-phosphoglycerol (DMPG), 1,2-dipalmitoyl-sn-glycero-3-phosphoglycerol (DPPG), 1-palmitoyl-2-oleoyl-sn-glycero-3-phosphoglycerol (POPG), 1,2-dioleoyl-sn-glycero-3-phosphoglycerol (DOPG), 1-stearoyl-2-oleoyl-sn-glycero-3-phosphoglycerol (SOPG), 1,2-distearoyl-sn-glycero-3-phosphoglycerol (DSPG), 1,2-dipalmitoyl-sn-glycero-3-phosphoserine (DPPS), 1-palmitoyl-2-oleoyl-sn-glycero-3-phosphoserine (POPS), 1,2-dioleoyl-sn-glycero-3-phosphoserine (DOPS), 1,2-dilinoleoyl-sn-glycero-3-phosphoserine (DLoPS), 1-stearoyl-2-oleoyl-sn-glycero-3-phosphoserine (SOPS), and 1,2-distearoyl-sn-glycero-3-phosphoserine (DSPS).
[4] The lipid nanoparticle of any one of [1] to [3], wherein the aforementioned anionic phospholipid is 1-palmitoyl-2-oleoyl-sn-glycero-3-phosphoglycerol (POPG), 1,2-dioleoyl-sn-glycero-3-phosphoglycerol (DOPG), or 1,2-dilinoleoyl-sn-glycero-3-phosphoserine (DLoPS).
[5] The lipid nanoparticle of any one of [1] to [4], wherein T in the formula (2) is a divalent aliphatic hydrocarbon group having 1 - 3 carbon atoms.
[6] The lipid nanoparticle of any one of [1] to [5], wherein the ionic lipid represented by the formula (1) is an ionic lipid represented by either the following formula: 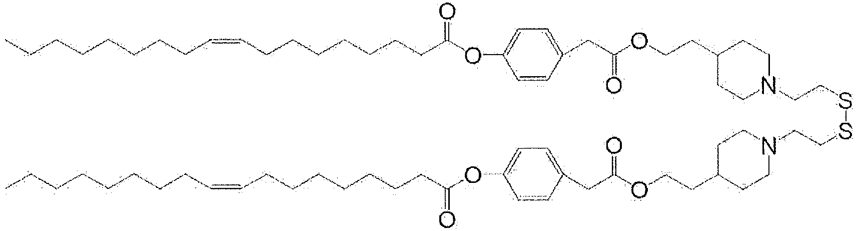 or 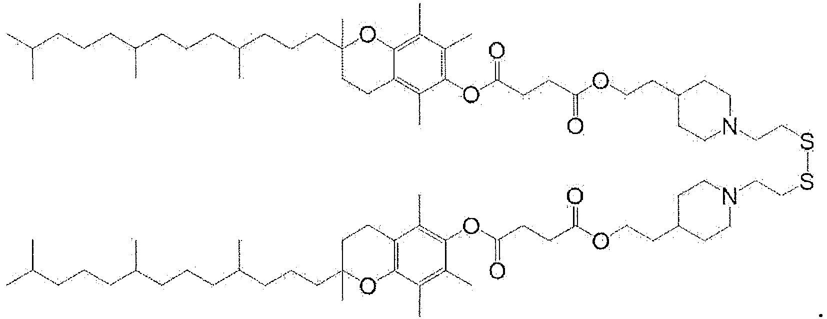
[7] The lipid nanoparticle of any one of [1] to [6], wherein a neutral phospholipid is optionally comprised as a component of the aforementioned lipid nanoparticle.
[8] The lipid nanoparticle of [7], wherein the aforementioned neutral phospholipid is 1,2-diacyl-sn-glycero-3-phosphocholine or 1,2-diacyl-sn-glycero-3-phosphoethanolamine.
[9] The lipid nanoparticle of [7] or [8], wherein the aforementioned neutral phospholipid is at least one selected from the group consisting of 1,2-dioleoyl-sn-glycero-3-phosphocholine (DOPC), 1,2-distearoyl-sn-glycero-3-phosphocholine (DSPC), 1,2-dierucoyl-sn-glycero-3-phosphocholine (DEPC), 1,2-dipalmitoyl-sn-glycero-3-phosphocholine (DPPC), 1-palmitoyl-2-oleoyl-sn-glycero-3-phosphocholine (POPC), 1-stearoyl-2-oleoyl-sn-glycero-3-phosphocholine (SOPC), 1,2-dioleoyl-sn-glycero-3-phosphoethanolamine (DOPE), and 1-palmitoyl-2-oleoyl-sn-glycero-3-phosphoethanolamine (POPE).
[10] The lipid nanoparticle of any one of [7] to [9], wherein a ratio of the aforementioned anionic phospholipid or the compound represented by the aforementioned formula (2), and the aforementioned neutral phospholipid is 100:0 to 25:75 mol%.
[11] The lipid nanoparticle of any one of [7] to [10], wherein the aforementioned ionic lipid is 30 to 70 mol%, the aforementioned anionic phospholipid or the aforementioned compound represented by the formula (2) is 2.5 to 15 mol%, the aforementioned neutral phospholipid is 0 to 15 mol%, the aforementioned cholesterol is 20 to 60 mol%, and the dimyristoylglycerol PEG is 0.5 to 1.5 mol%, relative to the total of the aforementioned ionic lipid, the aforementioned anionic phospholipid or the aforementioned compound represented by the formula (2), the aforementioned neutral phospholipid, and the aforementioned cholesterol.
[12] A method for delivering a nucleic acid to a spleen tissue, comprising intravenously administering the lipid nanoparticle of any one of [1] - [11] encapsulating the nucleic acid to a living organism.
[13] Use of a lipid nanoparticle comprising
   (A) an ionic lipid represented by the formula (1): 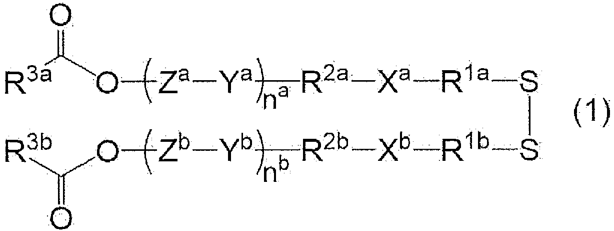 (in the formula (1), each symbol is as defined in [1]),
   (B) an anionic phospholipid or a compound represented by the formula (2): 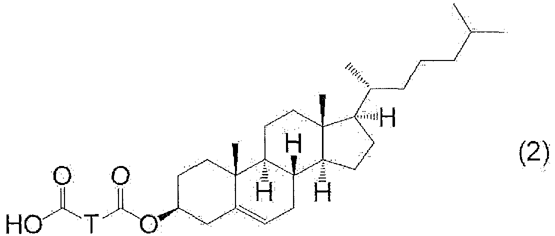 (in the formula (2), T is as defined in [1]),
   (C) cholesterol, and
   (D) a dimyristoylglycerol PEG represented by the formula (3):

      CH₂(OR⁶)-CH(OR⁷)-CH₂(OR⁸) (3)

      (in the formula (3), each symbol is as defined in [1])
      in the production of a medicament for delivering a nucleic acid to a spleen tissue.
[14] The use of [13], wherein the aforementioned anionic phospholipid is 1,2-diacyl-sn-glycero-3-phosphoglycerol or 1,2-diacyl-sn-glycero-3-phosphoserine.
[15] The use of [13] or [14], wherein the aforementioned anionic phospholipid is at least one selected from the group consisting of 1,2-dimyristoyl-sn-glycero-3-phosphoglycerol (DMPG), 1,2-dipalmitoyl-sn-glycero-3-phosphoglycerol (DPPG), 1-palmitoyl-2-oleoyl-sn-glycero-3-phosphoglycerol (POPG), 1,2-dioleoyl-sn-glycero-3-phosphoglycerol (DOPG), 1-stearoyl-2-oleoyl-sn-glycero-3-phosphoglycerol (SOPG), 1,2-distearoyl-sn-glycero-3-phosphoglycerol (DSPG), 1,2-dipalmitoyl-sn-glycero-3-phosphoserine (DPPS), 1-palmitoyl-2-oleoyl-sn-glycero-3-phosphoserine (POPS), 1,2-dioleoyl-sn-glycero-3-phosphoserine (DOPS), 1,2-dilinoleoyl-sn-glycero-3-phosphoserine (DLoPS), 1-stearoyl-2-oleoyl-sn-glycero-3-phosphoserine (SOPS), and 1,2-distearoyl-sn-glycero-3-phosphoserine (DSPS).
[16] The use of any one of [13] to [15], wherein the aforementioned anionic phospholipid is 1-palmitoyl-2-oleoyl-sn-glycero-3-phosphoglycerol (POPG), 1,2-dioleoyl-sn-glycero-3-phosphoglycerol (DOPG), or 1,2-dilinoleoyl-sn-glycero-3-phosphoserine (DLoPS).
[17] The use of any one of [13] to [16], wherein T in the formula (2) is a divalent aliphatic hydrocarbon group having 1 - 3 carbon atoms.
[18] The use of any one of [13] to [17], wherein the ionic lipid represented by the formula (1) is an ionic lipid represented by either the following formula: 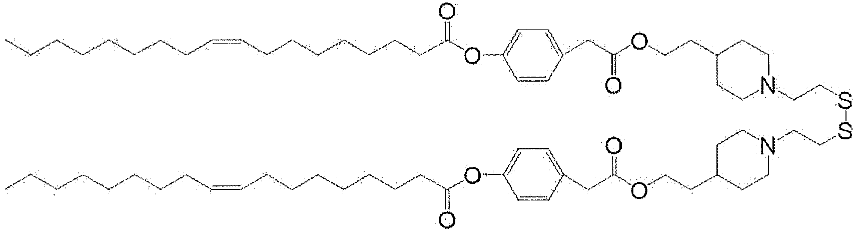 or 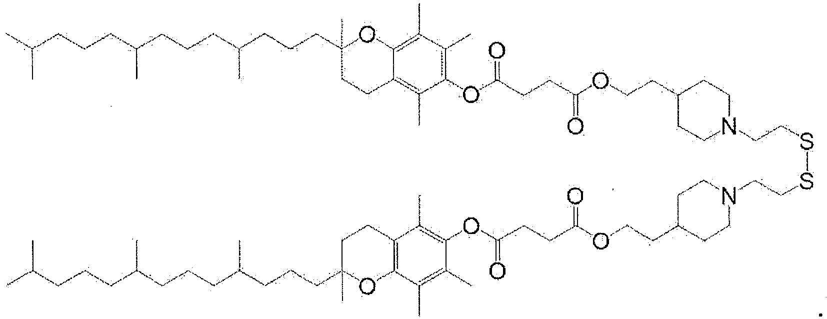
[19] The use of any one of [13] to [18], wherein a neutral phospholipid is optionally comprised as a component of the aforementioned lipid nanoparticle.
[20] The use of [19], wherein the aforementioned neutral phospholipid is 1,2-diacyl-sn-glycero-3-phosphocholine or 1,2-diacyl-sn-glycero-3-phosphoethanolamine.
[21] The use of [19] or [20], wherein the aforementioned neutral phospholipid is at least one selected from the group consisting of 1,2-dioleoyl-sn-glycero-3-phosphocholine (DOPC), 1,2-distearoyl-sn-glycero-3-phosphocholine (DSPC), 1,2-dierucoyl-sn-glycero-3-phosphocholine (DEPC), 1,2-dipalmitoyl-sn-glycero-3-phosphocholine (DPPC), 1-palmitoyl-2-oleoyl-sn-glycero-3-phosphocholine (POPC), 1-stearoyl-2-oleoyl-sn-glycero-3-phosphocholine (SOPC), 1,2-dioleoyl-sn-glycero-3-phosphoethanolamine (DOPE), and 1-palmitoyl-2-oleoyl-sn-glycero-3-phosphoethanolamine (POPE).
[22] The use of any one of [19] to [21], wherein a ratio of the aforementioned anionic phospholipid or the compound represented by the aforementioned formula (2), and the aforementioned neutral phospholipid is 100:0 to 25:75 mol%.
[23] The use of any one of [19] to [22], wherein the aforementioned ionic lipid is 30 to 70 mol%, the aforementioned anionic phospholipid or the aforementioned compound represented by the formula (2) is 2.5 to 15 mol%, the aforementioned neutral phospholipid is 0 to 15 mol%, the aforementioned cholesterol is 20 to 60 mol%, and the dimyristoylglycerol PEG is 0.5 to 1.5 mol%, relative to the total of the aforementioned ionic lipid, the aforementioned anionic phospholipid or the aforementioned compound represented by the formula (2), the aforementioned neutral phospholipid, and the aforementioned cholesterol.

### [Advantageous Effects of Invention]

The lipid nanoparticles of the present invention can deliver nucleic acids to spleen tissue more efficiently than lipid compositions of the prior art, by combining an ionic lipid that has a pKa suitable for endosomal escape and that is specifically decomposed in a reductive environment within a cell, and an anionic phospholipid or an anionic cholesterol, and by optimizing the lipid composition.

### [Brief Description of Drawings]

[Fig. 1]

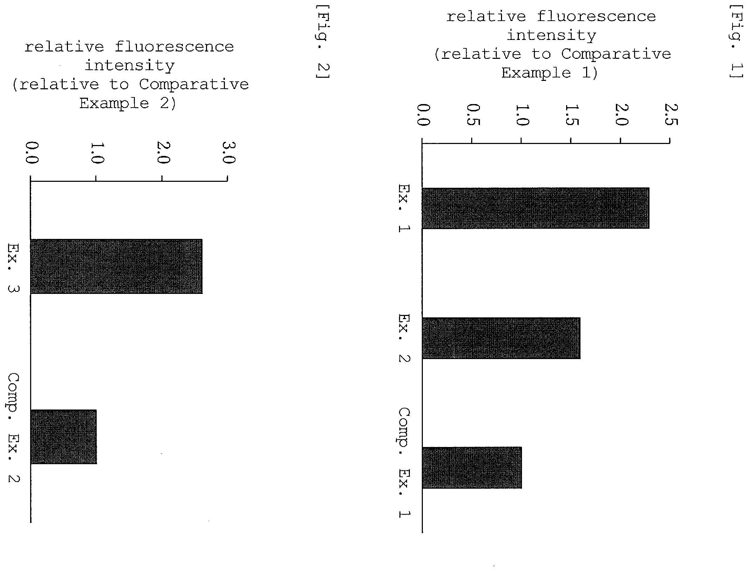

   Fig. 1 is a graph showing gene expression activity in the spleen of the lipid nanoparticles (LNPs) of Examples 1 and 2, and the LNP of Comparative Example 1, which is a composition for liver transfer.
[Fig. 2]
   Fig. 2 is a graph showing accumulation of LNP in the spleen of the LNP of Example 3, and the LNP of Comparative Example 2, which is a composition for liver transfer.
[Fig. 3]

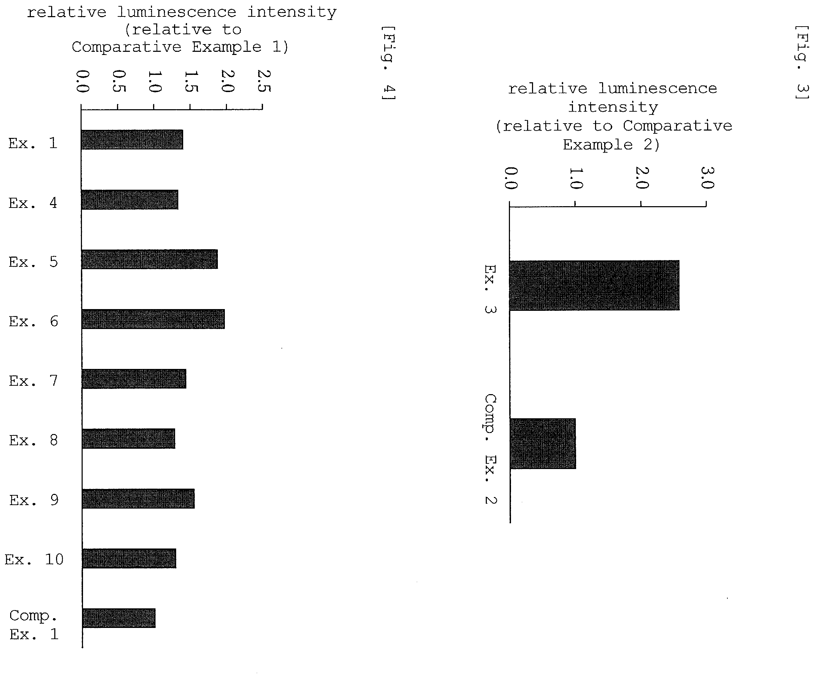

   Fig. 3 is a graph showing gene expression activity in the spleen of the LNP of Example 3, and the LNP of Comparative Example 2, which is a composition for liver transfer.
[Fig. 4]
   Fig. 4 is a graph showing gene expression activity in the spleen of the LNPs of Examples 1 and 4 to 10, and the LNP of Comparative Example 1, which is a composition for liver transfer.
[Fig. 5]

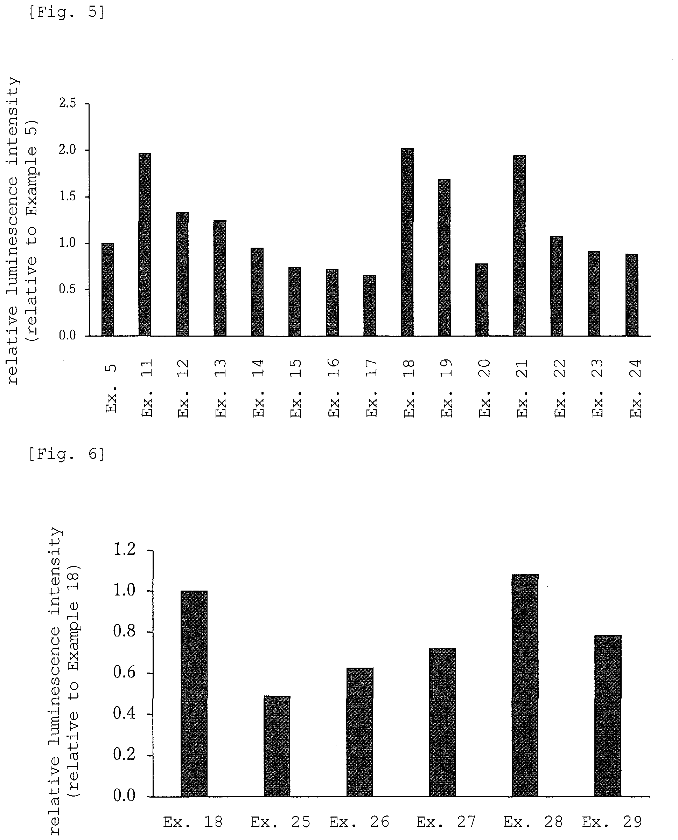

   Fig. 5 is a graph showing gene expression activity in the spleen of the LNPs of Examples 5 and 11 to 24.
[Fig. 6]
   Fig. 6 is a graph showing gene expression activity in the spleen of the LNPs of Examples 18 and 25 to 29.
[Fig. 7]

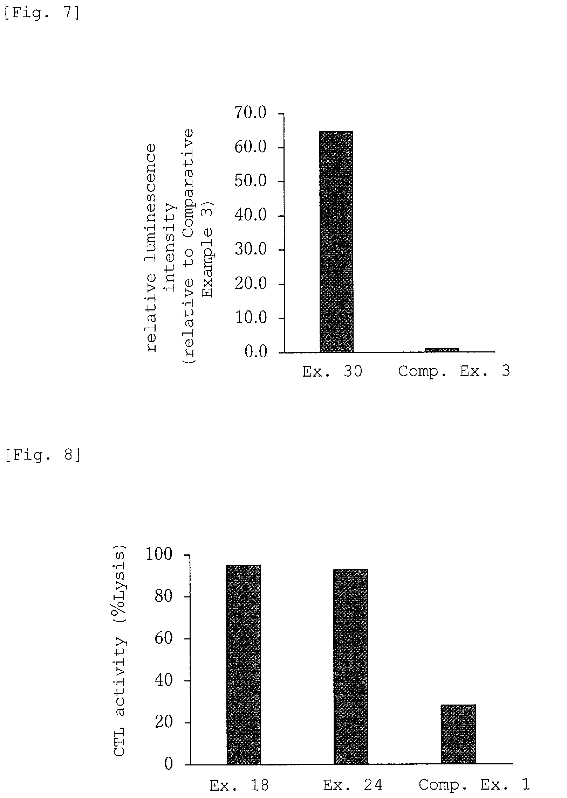

   Fig. 7 is a graph showing gene expression activity in the spleen of the LNP of Example 30, and the LNP of Comparative Example 3, which is a composition for delivery to the liver.
[Fig. 8]
   Fig. 8 is a graph showing CTL activity when the LNPs of Examples 18 and 24 encapsulating OVA-mRNA and the LNP of Comparative Example 1, which is a composition for delivery to the liver, were administered to mice.
[Fig. 9]

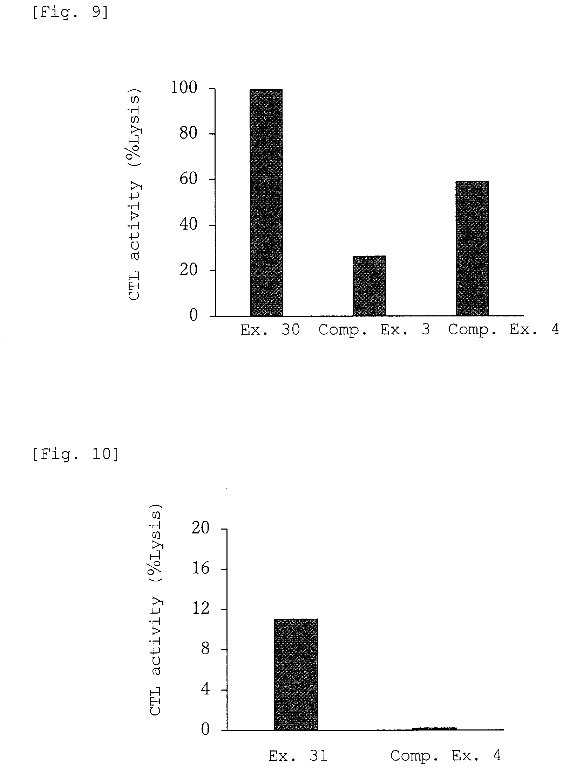

   Fig. 9 is a graph showing CTL activity when the LNP of Example 30 encapsulating OVA-mRNA, the LNP of Comparative Example 3, which is a composition for delivery to the liver, and the LNP of Comparative Example 4, which is a composition for delivery to the spleen, were administered to mice.
[Fig. 10]
   Fig. 10 is a graph showing CTL activity when the LNP of Example 31 encapsulating OVA-mRNA, and the LNP of Comparative Example 4, which is a composition for delivery to the spleen, were administered to mice.
[Fig. 11]

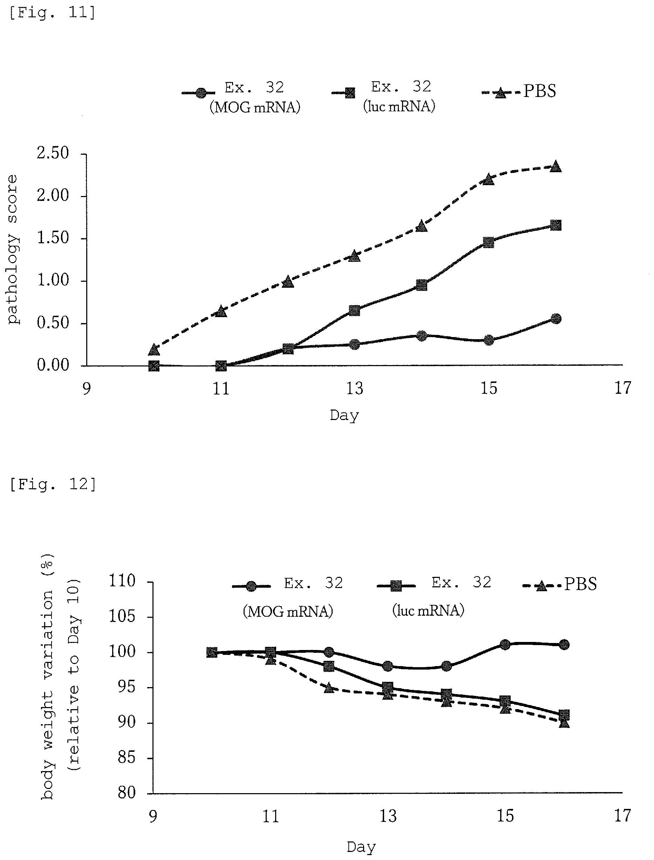

   Fig. 11 is a graph showing pathology scores of mice administered with the LNP of Example 32 encapsulating MOG-mRNA or luc-mRNA, or PBS.
[Fig. 12]
   Fig. 12 is a graph showing body weight variation (%) (relative to Day 10) of mice administered with the LNP of Example 32 encapsulating MOG-mRNA or luc-mRNA, or PBS.

### [Description of Embodiments]

While the embodiments of the present invention are explained in the following, the present invention is not limited thereto. The present invention relates to lipid nanoparticles containing an ionic lipid represented by the formula (1) (i.e., ionic lipid having a tertiary amino group, a lipid moiety, and a disulfide bond which is a biodegradable group), an anionic phospholipid or an anionic cholesterol represented by the formula (2), cholesterol, and a dimyristoylglycerol PEG represented by the formula (3), and a method for delivering nucleic acids to spleen cells.

### Lipid nanoparticles

In the present specification, the "lipid nanoparticle" (sometimes to be abbreviated as "LNP" in the present specification) means a particle having a membrane structure wherein the hydrophilic groups of amphiphilic lipid are arranged in the interface, facing the aqueous phase side and having a particle size of less than 1 µm, and the "amphiphilic lipid" means a lipid having both a hydrophilic group and a hydrophobic group.

The particle size of the lipid nanoparticles of the present invention is preferably 10 nm - 500 nm, more preferably 30 nm - 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present specification, the "particle size" means an average particle size (zeta mean) measured by a dynamic light scattering method.

Examples of the amphiphilic lipid include ionic lipid, phospholipid, PEG lipid, and the like. In the present specification, "PEG" means polyethylene glycol, "PEG lipid" means a lipid modified with PEG, and "Y modified with X" (e.g., X:PEG, Y:lipid) means Y bound to X. In other words, "PEG lipid" means a lipid bound to PEG.

The lipid nanoparticles of the present invention may contain lipids other than the ionic lipid represented by the formula (1), anionic phospholipid or anionic cholesterol represented by the formula (2), neutral phospholipid, cholesterol, and dimyristoylglycerol PEG represented by the formula (3) (hereinafter sometimes referred to as "other lipids"). Examples of other lipids include sterols other than cholesterol and PEG lipids other than dimyristoylglycerol PEG represented by the formula (3).

The amount of other lipids in the lipid nanoparticles of the present invention is preferably 0 to 50 mol%, more preferably 0 to 30 mol%, further preferably 0 to 10 mol%, relative to the total amount of lipids in the lipid nanoparticles. As used herein, the "total amount of lipids in the lipid nanoparticles" means, for example, when the lipid nanoparticles contain an ionic lipid represented by the formula (1), a phospholipid, cholesterol, a dimyristoylglycerol PEG represented by the formula (3), and other lipids as constituent components, the "total amount of the ionic lipid represented by the formula (1), phospholipid, cholesterol, dimyristoylglycerol PEG represented by the formula (3), and other lipids". In the present specification, the "amount of B relative to A (mol%)" means "100 x amount of B (mol)/amount of A (mol)". For example, the "amount of other lipids relative to the total amount of lipids (mol%)" means "100 x amount of other lipids (mol)/total amount of lipids (mol)".

In the present invention, it is most preferred to use no other lipids, that is, the lipids constituting the lipid nanoparticles of the present invention consist of an ionic lipid represented by the formula (1), anionic phospholipid or an anionic cholesterol represented by the formula (2), neutral phospholipid, cholesterol, and a dimyristoylglycerol PEG represented by the formula (3).

### Ionic lipid

The ionic lipid used in the present invention is an ionic lipid represented by the following formula (1) (sometimes abbreviated as "ionic lipid (1)" in the present specification). Only one kind of ionic lipid (1) may be used, or two or more kinds thereof may be used in combination. 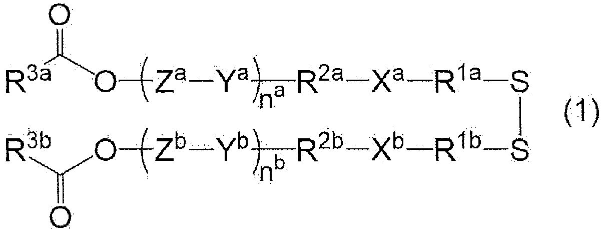 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

   R⁹-O-CO-(CH₂)a- (4)

   (in the formula (4),
   R⁹ is an aliphatic hydrocarbon group having 2 - 20 carbon atoms, and
   a is an integer of 2 to 10)).

R^{1a} and R^{1b} are each independently an alkylene group having 1 - 6 carbon atoms, and may be linear or branched, preferably linear. The carbon number of the alkylene group is preferably 1 - 4, more preferably 1 - 2. Specific examples of the alkylene group having 1 - 6 carbon atoms include methylene group, ethylene group, trimethylene group, isopropylene group, tetramethylene group, isobutylene group, pentamethylene group, neopentylene group and the like. Preferably, R^{1a} and R^{1b} are each independently a methylene group, an ethylene group, a trimethylene group, an isopropylene group or a tetramethylene group, most preferably an ethylene group.

R^{1a} may be the same as or different from R^{1b}, and R^{1a} is preferably the same group as R^{1b}.

X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups, preferably each independently a cyclic alkylene tertiary amino group having 2 - 5 carbon atoms and 1 - 2 tertiary amino groups.

The alkyl group having 1 - 6 carbon atoms in the non-cyclic alkyl tertiary amino group having 1 - 6 carbon atoms and one tertiary amino group may be linear, branched or cyclic. The alkyl group preferably has a carbon number of 1 - 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, t-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like, preferably methyl group, ethyl group, propyl group or isopropyl group, most preferably methyl group.

A preferable specific structure of the non-cyclic alkyl tertiary amino group having 1 - 6 carbon atoms and one tertiary amino group is represented by X¹.

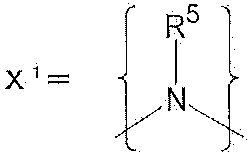

R⁵ in X¹ is an alkyl group having 1 - 6 carbon atoms, which may be linear, branched or cyclic. The alkyl group preferably has a carbon number of 1 - 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. R⁵ is preferably methyl group, ethyl group, propyl group or isopropyl group, most preferably methyl group.

The carbon number of the cyclic alkylene tertiary amino group having 2 - 5 carbon atoms and 1 - 2 tertiary amino groups is preferably 4 - 5. The cyclic alkylene tertiary amino group having 2 - 5 carbon atoms and 1 - 2 tertiary amino groups is specifically aziridylene group, azetidylene group, pyrrolidylene group, piperidylene group, imidazolidylene group, or piperazylene group, preferably pyrrolidylene group, piperidylene group, or piperazylene group, most preferably piperidylene group.

A preferable specific structure of the cyclic alkylene tertiary amino group having 2 - 5 carbon atoms and one tertiary amino group is represented by X².

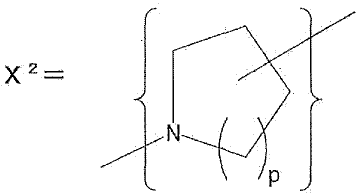

The p of X² is 1 or 2. When p is 1, X² is a pyrrolidylene group, and when p is 2, X² is a piperidylene group. Preferably, p is 2.

A preferable specific structure of the cyclic alkylene tertiary amino group having 2 - 5 carbon atoms and two tertiary amino groups is represented by X³.

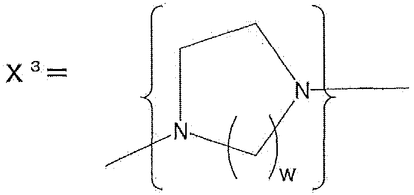

The w of X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group and the like, preferably methylene group, ethylene group, trimethylene group, and tetramethylene group, most preferably ethylene group.

In the present specification, the "oxydialkylene group having not more than 8 carbon atoms" means a group containing alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group) and the like. Preferably, it is oxydimethylene group, oxydiethylene group, oxydi(trimethylene) group, most preferably oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 - 16 carbon atoms and at least one aromatic ring, and optionally having a hetero atom. The number of carbons contained in the aromatic compound is preferably 6 - 12, most preferably 6 - 7. The aromatic ring contained in the aromatic compound is preferably one.

As the kind of the aromatic ring contained in the aromatic compound having 3 - 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring and the like can be mentioned for aromatic heterocycle. It is preferably benzene ring, naphthalene ring, or anthracene ring, most preferably benzene ring.

The aromatic ring may have a substituent. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like.

A preferable specific structure of Z^{a} and Z^{b} is Z¹.

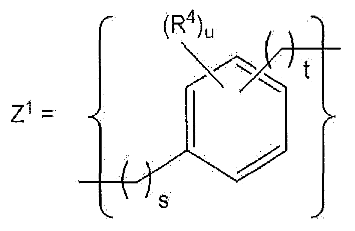

wherein s is an integer of 0 - 3, t is an integer of 0 - 3, u is an integer of 0 - 4, and R⁴ in the number of u are each independently a substituent.

The s for Z¹ is preferably an integer of 0 - 1, more preferably 0.

The t for Z¹ is preferably an integer of 0 - 2, more preferably 1.

The u for Z¹ is preferably an integer of 0 - 2, more preferably an integer of 0 - 1.

The R⁴ for Z¹ is a substituent of an aromatic ring (benzene ring) contained in the aromatic compound having 3 - 16 carbon atoms which does not inhibit the reaction in the synthesis process of an ionic lipid. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

R⁹-O-CO-(CH₂)a- (4)

(in the formula (4),
R⁹ is an aliphatic hydrocarbon group having 2 - 20 carbon atoms, and
a is an integer of 2 to 10), preferably each independently a residue derived from a reaction product of a liposoluble vitamin having a hydroxyl group, and succinic anhydride or glutaric anhydride, or an aliphatic hydrocarbon group having 12 - 22 carbon atoms, most preferably each independently an aliphatic hydrocarbon group having 12 - 22 carbon atoms.

The residue derived from a reaction product of a liposoluble vitamin having a hydroxyl group, and succinic anhydride or glutaric anhydride is a group having a structure in which the hydroxyl group of a liposoluble vitamin having a hydroxyl group is replaced by *-O-CO-CH₂-CH₂- or *-O-CO-CH₂-CH₂-CH₂-. * is the bonding position with the liposoluble vitamin. The residue derived from a reaction product of a sterol derivative having a hydroxyl group, and succinic anhydride or glutaric anhydride is a group having a structure in which the hydroxyl group of a sterol derivative having a hydroxyl group is replaced by *-O-CO-CH₂-CH₂- or *-O-CO-CH₂-CH₂-CH₂-. * is the bonding position with the sterol derivative.

The liposoluble vitamin having a hydroxyl group is, for example, retinol, ergosterol, 7-dehydrocholesterol, calciferol, cholecalciferol, dihydroergocalciferol, dihydrotachysterol, tocopherol, tocotrienol and the like. Preferred example of the liposoluble vitamin having a hydroxyl group is tocopherol.

Examples of the sterol derivative having a hydroxyl group include cholesterol, cholestanol, stigmasterol, β-sitosterol, lanosterol, and ergosterol and the like, preferably cholesterol or cholestanol.

The aliphatic hydrocarbon group having 1 - 40 carbon atoms may be linear or branched. The aliphatic hydrocarbon group may be saturated or unsaturated. In the case of an unsaturated aliphatic hydrocarbon group, the aliphatic hydrocarbon group generally contains 1 - 6, preferably 1 - 3, more preferably 1 - 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 - 22, more preferably 13 - 19, most preferably 13 - 17. While the aliphatic hydrocarbon group includes an alkyl group, an alkenyl group, an alkynyl group and the like, it is preferably an alkyl group or an alkenyl group. Specific examples of the aliphatic hydrocarbon group having 1 - 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group and the like. The aliphatic hydrocarbon group having 1 - 40 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In one embodiment of the present invention, the aliphatic hydrocarbon group having 1 - 40 (preferably 12 - 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, and heptadecenyl group when oleic acid is used as the fatty acid.

The alkyl group having a cyclopropane ring and having 3 - 40 carbon atoms for R^{3a} or R^{3b} means an alkyl group having at least one cyclopropane ring in the alkyl chain and having 3 - 40 carbon atoms. The number of carbon atoms in the alkyl group is 3 to 40, and does not include the number of carbon atoms in the cyclopropane ring. The number of cyclopropane rings in the alkyl group is preferably one. The alkyl group having a cyclopropane ring and having 3 - 40 carbon atoms for R^{3a} or R^{3b} is preferably a group represented by the formula (5): 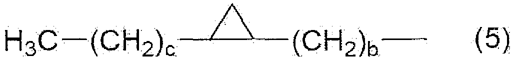 (in the formula (5), b and c are each independently an integer and the total of b and c is 2 to 39). Preferably, b is an integer of 1 to 20, c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (5) include 7-(2-octylcyclopropyl)heptyl and the like.

In the formula (4), the aliphatic hydrocarbon group having 2 - 20 carbon atoms for R⁹ may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably alkyl group. Specific examples of the aliphatic hydrocarbon group having 2 to 20 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 - 20 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In the formula (4), a is preferably an integer of 3 to 9, more preferably an integer of 3 to 7, further preferably an integer of 5 to 7, and most preferably 5 or 7.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferable examples of ionic lipid (1) include the following ionic lipids.

### [Ionic lipid (1-1)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 - 2 carbon atoms (i.e., methylene group or ethylene group);
X^{a} and X^{b} are each independently X¹: 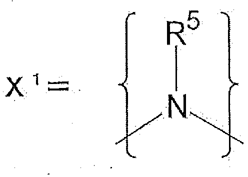 wherein R⁵ is an alkyl group having 1 - 3 carbon atoms (e.g., methyl group), or X²: 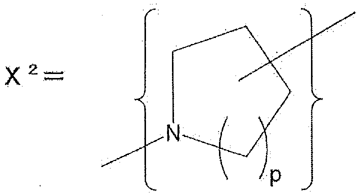 wherein p is 1 or 2;
   R^{2a} and R^{2b} are each independently alkylene group having not more than 4 carbon atoms (e.g., methylene group, ethylene group, trimethylene group);
   Y^{a} and Y^{b} are each independently an ester bond or an amide bond;
   Z^{a} and Z^{b} are each independently Z¹: 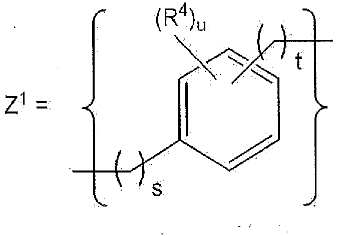
   wherein s is an integer of 0 - 1, t is an integer of 0 - 2, u is an integer of 0 - 2 (preferably 0), and R⁴ in the number of u are each independently a substituent; and
   n^{a} and n^{b} are each independently 0 or 1; and
   R^{3a} and R^{3b} are each independently a residue derived from a reaction product of a liposoluble vitamin having a hydroxyl group (e.g., tocopherol) and succinic anhydride, or an aliphatic hydrocarbon group having 13 - 17 carbon atoms (e.g., heptadecenyl group, heptadecadienyl group, 1-hexylnonyl group).

Specific examples of the ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, O-Ph-C3M, and TS-P4C2.

Specific examples of ionic lipid (1) are Lipid 1 to Lipid 20 described in WO 2021/195529 A2. Particularly, the following Lipid 1, Lipid 5, and Lipid 8 can be mentioned.

**[Table 1-1]**

| name of ionic lipid | structure |
|---|---|
| O-Ph-P3C1 | 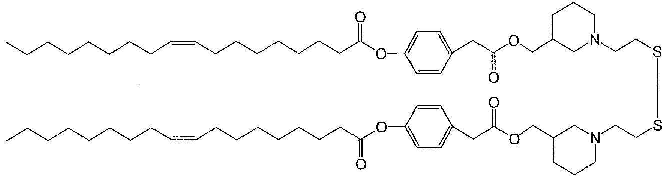 |
| O-Ph-P4C1 | 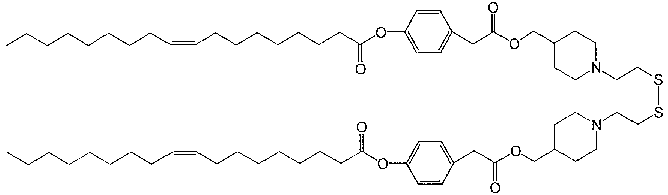 |
| O-Ph-P4C2 (or SS-OP) | 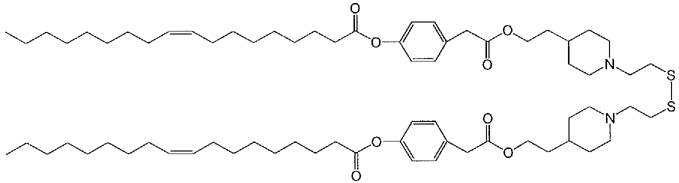 |
| O-Bn-P4C2 | 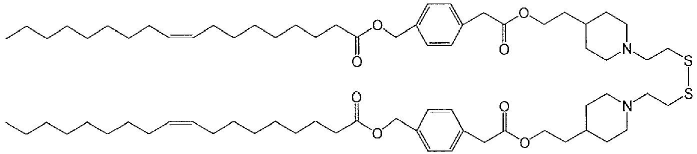 |
| E-Ph-P4C2 (or SS-EP) | 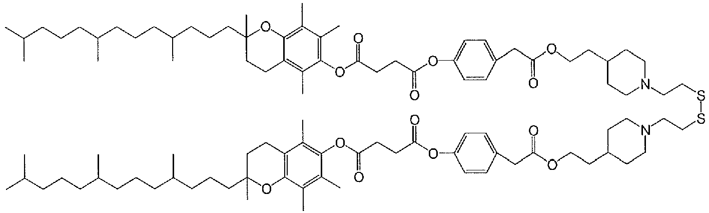 |

**[Table 1-2]**

| name of ionic lipid | structure |
|---|---|
| L-Ph-P4C2 | 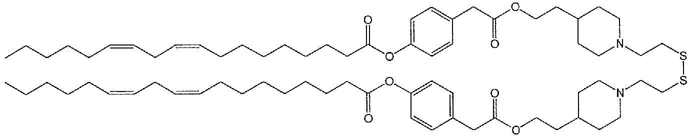 |
| HD-Ph-P4C2 | 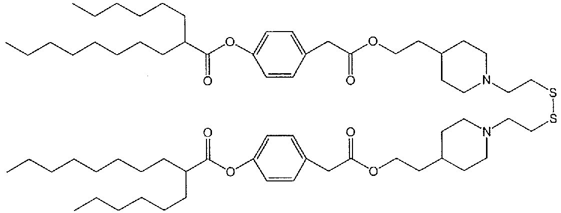 |
| O-Ph-amide-P4C2 | 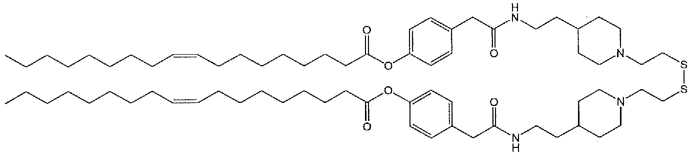 |
| O-Ph-C3M | 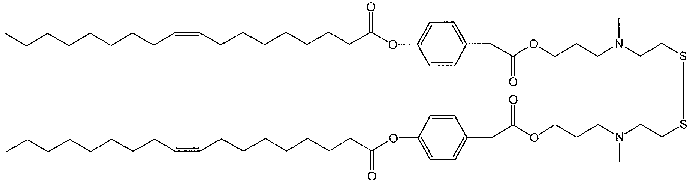 |
| TS-P4C2 (or SS-EC) | 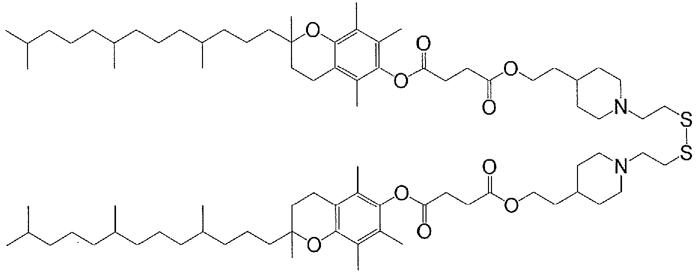 |

**[Table 1-3]**

| name of ionic lipid | structure |
|---|---|
| Lipid 1 | 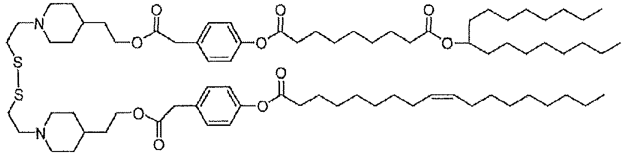 |
| Lipid 5 | 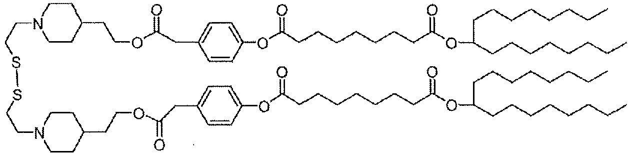 |
| Lipid 8 | 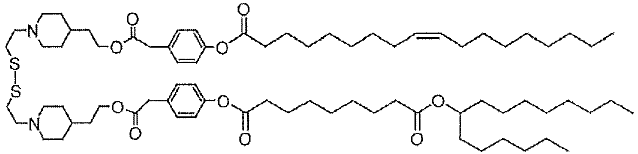 |

Among specific examples of ionic lipid (1), SS-OP or SS-EC is preferred. That is, ionic lipid (1) is preferably an ionic lipid represented by either the following formulas:

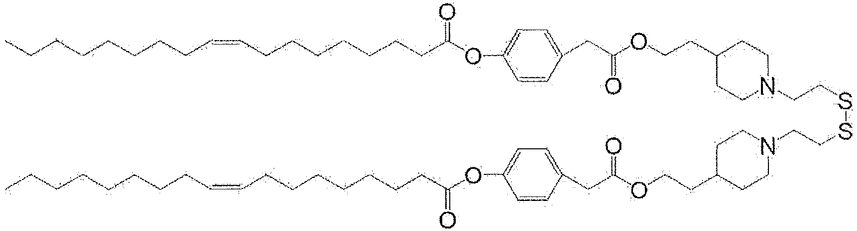

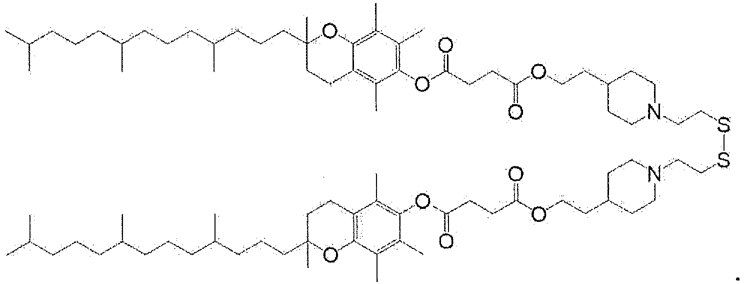

The amount of ionic lipid (1) in the lipid nanoparticles of the present invention is preferably 30 to 70 mol%, more preferably 40 to 65 mol%, further preferably 50 to 60 mol%, relative to the total of ionic lipid (1), anionic phospholipid or a compound represented by the formula (2), neutral phospholipid, and cholesterol, from the aspects of encapsulation efficiency of nucleic acid, release efficiency of nucleic acid in cells, and stability of lipid nanoparticles.

Ionic lipid (1) can be produced by a known method (e.g., methods described in WO 2019/188867 A1, US 9708628 B2, WO 2021/195529 A2).

### Phospholipid

The lipid nanoparticles of the present invention contain anionic phospholipid as phospholipid. The phospholipids may be anionic phospholipid alone, or anionic phospholipid and other neutral phospholipid may be used in combination.

Specific examples of the anionic phospholipid include 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), lyso forms of these, and the like.

Specific examples of 1,2-diacyl-sn-glycero-3-phosphoserine (PS) include 1,2-didecanoyl-sn-glycero-3-phosphoserine (DDPS), 1,2-dilauroyl-sn-glycero-3-phosphoserine (DLPS), 1,2-dimyristoyl-sn-glycero-3-phosphoserine (DMPS), 1,2-dipalmitoyl-sn-glycero-3-phosphoserine (DPPS), 1,2-distearoyl-sn-glycero-3-phosphoserine (DSPS), 1,2-dioleoyl-sn-glycero-3-phosphoserine (DOPS), 1,2-dilinoleoyl-sn-glycero-3-phosphoserine (DLoPS), 1,2-dierucoyl-sn-glycero-3-phosphoserine (DEPS), 1-myristoyl-2-palmitoyl-sn-glycero-3-phosphoserine (MPPS), 1-myristoyl-2-stearoyl-sn-glycero-3-phosphoserine (MSPS), 1-palmitoyl-2-myristoyl-sn-glycero-3-phosphoserine (PMPS), 1-palmitoyl-2-stearoyl-sn-glycero-3-phosphoserine (PSPS), 1-palmitoyl-2-oleoyl-sn-glycero-3-phosphoserine (POPS), and 1-stearoyl-2-oleoyl-sn-glycero-3-phosphoserine (SOPS). In the present specification, phospholipids may be referred to by their abbreviations. For example, 1,2-diacyl-sn-glycero-3-phosphoserine may be referred to as PS, and 1,2-didecanoyl-sn-glycero-3-phosphoserine may be referred to as DDPS.

Specific examples of 1,2-diacyl-sn-glycero-3-phosphoglycerol (PG) include 1,2-didecanoyl-sn-glycero-3-phosphoglycerol (DDPG), 1,2-dilauroyl-sn-glycero-3-phosphoglycerol (DLPG), 1,2-dimyristoyl-sn-glycero-3-phosphoglycerol (DMPG), 1,2-dipalmitoyl-sn-glycero-3-phosphoglycerol (DPPG), 1,2-distearoyl-sn-glycero-3-phosphoglycerol (DSPG), 1,2-dioleoyl-sn-glycero-3-phosphoglycerol (DOPG), 1,2-dilinoleoyl-sn-glycero-3-phosphoglycerol (DLoPG), 1,2-dierucoyl-sn-glycero-3-phosphoglycerol (DEPG), 1-myristoyl-2-palmitoyl-sn-glycero-3-phosphoglycerol (MPPG), 1-myristoyl-2-stearoyl-sn-glycero-3-phosphoglycerol (MSPG), 1-palmitoyl-2-myristoyl-sn-glycero-3-phosphoglycerol (PMPG), 1-palmitoyl-2-stearoyl-sn-glycero-3-phosphoglycerol (PSPG), 1-palmitoyl-2-oleoyl-sn-glycero-3-phosphoglycerol (POPG), and 1-stearoyl-2-oleoyl-sn-glycero-3-phosphoglycerol (SOPG).

Specific examples of 1,2-diacyl-sn-glycero-3-phosphatidic acid (PA) include 1,2-didecanoyl-sn-glycero-3-phosphatidic acid (DDPA), 1,2-dilauroyl-sn-glycero-3-phosphatidic acid (DLPA), 1,2-dimyristoyl-sn-glycero-3-phosphatidic acid (DMPA), 1,2-dipalmitoyl-sn-glycero-3-phosphatidic acid (DPPA), 1,2-distearoyl-sn-glycero-3-phosphatidic acid (DSPA), 1,2-dioleoyl-sn-glycero-3-phosphatidic acid (DOPA), 1,2-dilinoleoyl-sn-glycero-3-phosphatidic acid (DLoPA), 1,2-dierucoyl-sn-glycero-3-phosphatidic acid (DEPA), 1-myristoyl-2-palmitoyl-sn-glycero-3-phosphatidic acid (MPPA), 1-myristoyl-2-stearoyl-sn-glycero-3-phosphatidic acid (MSPA), 1-palmitoyl-2-myristoyl-sn-glycero-3-phosphatidic acid (PMPA), 1-palmitoyl-2-stearoyl-sn-glycero-3-phosphatidic acid (PSPA), 1-palmitoyl-2-oleoyl-sn-glycero-3-phosphatidic acid (POPA), and 1-stearoyl-2-oleoyl-sn-glycero-3-phosphatidic acid (SOPA).

The anionic phospholipid is preferably 1,2-diacyl-sn-glycero-3-phosphoglycerol, more preferably at least one selected from the group consisting of DMPG, DPPG, POPG, DOPG, SOPG and DSPG, further preferably POPG or DOPG. These phospholipids have similar acyl chain structures and are therefore considered to afford similar effects.

Other forms of anionic phospholipid include preferably 1,2-diacyl-sn-glycero-3-phosphoserine, more preferably at least one selected from the group consisting of DPPS, POPS, DOPS, DLoPS, SOPS and DSPS, further preferably DLoPS. These phospholipids have similar acyl chain structures and are therefore considered to afford similar effects.

Other examples of neutral phospholipid include 1,2-diacyl-sn-glycero-3-phosphocholine (PC), 1,2-diacyl-sn-glycero-3-phosphoethanolamine (PE), the lyso forms of these, and the like.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-diarachidoyl-sn-glycero-3-phosphocholine (DAPC) 1,2-dieicosenoyl-sn-glycero-3-phosphocholine (DEiPC), 1,2-dibehenoyl-sn-glycero-3-phosphocholine (DBPC), 1,2-dierucoyl-sn-glycero-3-phosphocholine (DEPC), 1,2-dilignoceroyl-sn-glycero-3-phosphocholine (DLiPC), 1,2-dinervonoyl-sn-glycero-3-phosphocholine (DNPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), and 1-stearoyl-2-oleoyl-sn-glycero-3-phosphocholine (SOPC).

Specific examples of 1,2-diacyl-sn-glycero-3-phosphoethanolamine (PE) include 1,2-didecanoyl-sn-glycero-3-phosphoethanolamine (DOPE), 1,2-dilauroyl-sn-glycero-3-phosphoethanolamine (DLPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), 1,2-dioleoyl-sn-glycero-3-phosphoethanolamine (DOPE), 1,2-dilinoleoyl-sn-glycero-3-phosphoethanolamine (DLoPE), 1,2-dierucoyl-sn-glycero-3-phosphoethanolamine (DEPE), 1-myristoyl-2-palmitoyl-sn-glycero-3-phosphoethanolamine (MPPE), 1-myristoyl-2-stearoyl-sn-glycero-3-phosphoethanolamine (MSPE), 1-palmitoyl-2-myristoyl-sn-glycero-3-phosphoethanolamine (PMPE), 1-palmitoyl-2-stearoyl-sn-glycero-3-phosphoethanolamine (PSPE), 1-palmitoyl-2-oleoyl-sn-glycero-3-phosphoethanolamine (POPE), and 1-stearoyl-2-oleoyl-sn-glycero-3-phosphoethanolamine (SOPE).

The other neutral phospholipid is not particularly limited and is preferably at least one selected from the group consisting of DPPC, DOPC, DSPC, DEPC, POPC, SOPC, POPE and DOPE, more preferably at least one selected from the group consisting of DSPC, DOPC and DEPC, most preferably DEPC.

When the anionic phospholipid and other neutral phospholipid are use in combination, the ratio of the anionic phospholipid and the neutral phospholipid is preferably 100:0 to 25:75 mol%, more preferably 75:25 to 25:75 mol%, further preferably 50:50 mol%.

The total amount of anionic phospholipids in the lipid nanoparticles of the present invention is preferably 1 to 20 mol%, more preferably 2.5 to 15 mol%, further preferably 2.5 to 10 mol%, relative to the total of ionic lipid (1), anionic phospholipid or a compound represented by the formula (2), neutral phospholipid, and cholesterol, from the aspects of encapsulation efficiency of nucleic acid, release efficiency of nucleic acid in cells, and stability of lipid nanoparticles.

The total amount of neutral phospholipids in the lipid nanoparticles of the present invention is preferably 0 to 15 mol%, more preferably 2 to 10 mol%, further preferably 2.5 to 7.5 mol %, relative to the total of ionic lipid (1), anionic phospholipid or a compound represented by the formula (2), neutral phospholipid, and cholesterol, from the aspects of encapsulation efficiency of nucleic acid, release efficiency of nucleic acid in cells, and stability of lipid nanoparticles.

### Cholesterol

The lipid nanoparticles of the present invention may contain anionic cholesterol of the formula (2) (sometimes to be abbreviated as "anionic cholesterol (2)" in the present specification) instead of anionic phospholipid. 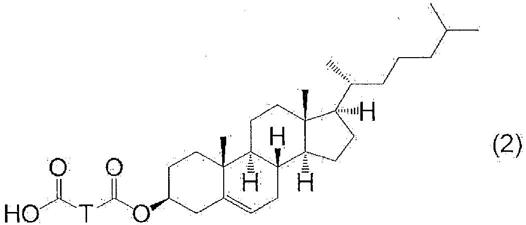 (in the formula (2),
T is a divalent aliphatic hydrocarbon group having 1 - 8 carbon atoms).

The divalent aliphatic hydrocarbon group having 1 - 8 carbon atoms represented by T may be linear or branched, or may contain unsaturation. T is preferably a linear divalent saturated aliphatic hydrocarbon group having 1 - 6 carbon atoms, more preferably a linear divalent saturated aliphatic hydrocarbon group having 1 - 3 carbon atoms. A specific example of anionic cholesterol (2) is cholesteryl hemisuccinate.

The total amount of anionic cholesterol (2) in the lipid nanoparticles of the present invention is preferably 1 to 20 mol%, more preferably 2.5 to 15 mol%, further preferably 2.5 to 10 mol%, relative to the total of ionic lipid (1), anionic phospholipid or anionic cholesterol (2), neutral phospholipid, and cholesterol, from the aspects of encapsulation efficiency of nucleic acid, release efficiency of nucleic acid in cells, and stability of lipid nanoparticles.

The lipid nanoparticles of the present invention includes cholesterol. The total amount of cholesterol in the lipid nanoparticles of the present invention is preferably 20 to 60 mol%, more preferably 25 to 50 mol%, further preferably 30 to 40 mol%, relative to the total of ionic lipid (1), anionic phospholipid or anionic cholesterol (2), neutral phospholipid, and cholesterol, from the aspects of encapsulation efficiency of nucleic acid, release efficiency of nucleic acid in cells, and stability of lipid nanoparticles.

### Dimyristoylglycerol PEG

The lipid nanoparticle of the present invention includes a dimyristoylglycerol PEG represented by the formula (3):

CH₂(OR⁶)-CH(OR⁷)-CH₂(OR⁸) (3)

(in the formula (3),
any two of R⁶, R⁷ and R⁸ are myristoyl groups, and the remaining one is an alkyl group having 1 - 6 carbon atoms connected via a polyethylene glycol chain (PEG chain) with a number average molecular weight of 1,000 to 3,000) (sometimes to be abbreviated as "dimyristoylglycerol PEG (3)" in the present specification).

The number average molecular weight of the PEG chain in the formula (3) is 1,000 to 3,000, preferably 1,500 to 2,500. The number average molecular weight of the PEG used to form the PEG chain can be measured by gel permeation chromatography (GPC) .

The alkyl group having 1 - 6 carbon atoms may be linear, branched or cyclic. The alkyl group preferably has a carbon number of 1 to 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. It is preferably a methyl group.

The amount of dimyristoylglycerol PEG (3) in the lipid nanoparticles of the present invention is preferably 0.5 - 1.5 mol%, more preferably 1.0 mol%, relative to the total amount of ionic lipid (1), anionic phospholipid or anionic cholesterol (2), neutral phospholipid, and cholesterol, from the aspects of nucleic acid encapsulation efficiency, intracellular nucleic acid release efficiency, and lipid nanoparticle stability.

### Optimal composition

The optimal molar ratio of ionic lipid (1):anionic phospholipid or anionic cholesterol (2):neutral phospholipid:cholesterol:dimyristoylglycerol PEG (3) in the lipid nanoparticles of the present invention is 55:5:5:35:1.0.

### Production method of lipid nanoparticles

The lipid nanoparticles of the present invention can be produced by dispersing lipid raw materials containing ionic lipid (1), anionic phospholipid or anionic cholesterol (2), cholesterol, and dimyristoylglycerol PEG (3) in an appropriate dispersion medium (e.g., aqueous dispersion medium, alcoholic dispersion medium), and performing an operation to induce organization as necessary.

Examples of the "operation to induce organization" for producing the lipid nanoparticles of the present invention include methods known per se such as an alcohol (e.g., ethanol, tert-butanol and the like) dilution method using a micro flow path or vortex, a simple hydration method, sonication, heating, vortex, an ether injecting method, a French press method, a cholic acid method, a Ca²⁺ fusion method, a freeze-thaw method, a reversed-phase evaporation method and the like, preferably an alcohol dilution method using a micro flow path or vortex, further preferably an alcohol dilution method using a micro flow path. In the alcohol dilution method using a micro flow path, for example, NanoAssemblr (registered trademark) (Precision NanoSystems) can be used to produce a dispersion containing lipid nanoparticles by mixing an acidic buffer containing nucleic acid with an ethanol solution of lipids. The dispersion produced by this method contains lipid nanoparticles and a dispersion medium (acidic buffer and alcohol), but removal of the dispersion medium (especially alcohol), exchange of the dispersion medium (particularly buffer), and the like can be performed by operations such as ultrafiltration, dialysis, dilution, and the like.

### Method for delivering nucleic acid to spleen cell

The present invention also provides a method for delivering nucleic acids to spleen cells including administering the lipid nanoparticles encapsulating nucleic acid of the present invention to a subject. In this method, lipid nanoparticles are preferably administered intravenously to the subject.

Examples of the nucleic acid include, but are not limited to, DNA, RNA, chimera nucleic acid of RNA, DNA/RNA hybrid and the like. While any nucleic acid having 1 to 3 chains can be used, it is preferably a single strand or double strand. The nucleic acid may be nucleotide having N-glycoside of purine or pyrimidine base or oligomer having a non-nucleotide backbone (e.g., commercially available peptide nucleic acid (PNA) etc.), oligomer containing a special bond (saidoligomer comprising base pairing or a nucleotide having a configuration permitting attachment of base, which are found in DNA and RNA) and the like.

Furthermore, it may be a nucleic acid added with known modification, for example, a nucleic acid with a label known in the field, a nucleic acid with a cap, a methylated nucleic acid, one or more natural nucleotides substituted by an analog, a nucleic acid with nucleotidyl modification, for example, a nucleic acid with non-charge bond (e.g., methylphosphonate, phosphotriester, phosphoramidate, carbamate and the like), a nucleic acid with a charged bond or sulfur-containing bond (e.g., phosphorothioate, phosphorodithioate and the like), for example, a nucleic acid with a side chain group such as protein (nuclease, nuclease inhibitor, toxin, antibody, signal peptide, poly-L-lysine and the like), sugar (e.g., monosaccharide and the like) and the like, a nucleic acid with an intercalating compound (e.g., acridine, psoralen and the like), a nucleic acid with a chelate compound (e.g., metal, radioactive metal, boron, oxidative metal and the like), a nucleic acid containing an alkylating agent, or a nucleic acid with a modified bond (e.g., α anomer-type nucleic acid and the like).

The type of the DNA that can be used in the present invention is not particularly limited, and can be selected as appropriate according to the purpose of use. As DNA, for example, plasmid DNA, cDNA, antisense DNA, chromosomal DNA, PAC, BAC, CpG oligosaccharide, and the like can be mentioned. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. As RNA, for example, siRNA, miRNA, shRNA, antisense RNA, messenger RNA (mRNA), single strand RNA genome, double strand RNA genome, RNA replicon, transfer RNA, ribosomal RNA and the like can be mentioned, with preference given to siRNA, miRNA, shRNA, mRNA, antisense RNA, and RNA replicon.

The nucleic acid used in the present invention is preferably purified by a method generally used by those of ordinary skill in the art.

The nucleic acid to be used in the present invention is preferably one having a preventive and/or therapeutic activity against a given disease (prophylactic/therapeutic nucleic acid). Examples of such nucleic acid include nucleic acids and the like used for so-called gene therapy.

The particle size of the lipid nanoparticles encapsulating nucleic acid of the present invention is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticles encapsulating the nucleic acid can be appropriately adjusted by the production method thereof.

The surface potential (zeta potential) of the lipid nanoparticles encapsulating the nucleic acid for introducing nucleic acid into spleen cells is preferably -30 to +10 mV, more preferably -25 to 0 mV. In conventional transgene, particles with positively charged surface have been mainly used. This is useful as a method for promoting electrostatic interactions with heparin sulfate on the negatively-charged cell surface to enhance uptake into cells. However, a positive surface potential can result in (a) inhibition of release of nucleic acid from the carrier through interaction with the delivered nucleic acid within cells, and (b) inhibition of protein synthesis through interaction between mRNA and the delivered nucleic acid. This problem can be solved by adjusting the surface potential (zeta potential) to fall within the above-mentioned range. The surface potential (zeta potential) can be measured using a zeta potential measuring apparatus such as Zetasizer Nano and the like. The surface potential (zeta potential) of the lipid nanoparticles can be adjusted by the composition of the constituent component of the lipid nanoparticles.

By administering the lipid nanoparticles of the present invention encapsulating nucleic acid to a living organism, the lipid nanoparticles reach and come into contact with spleen tissue, and the nucleic acid encapsulated in the lipid nanoparticles is delivered to the spleen tissue in the living organism. The target.to which the lipid nanoparticles can be administered are not particularly limited, and examples of such cells include cells of mammals (e.g., humans, monkeys, mice, rats, hamsters, cows, etc.), birds (e.g., chickens, ostriches, etc.), amphibians (e.g., frogs, etc.), and fish (e.g., zebrafish, rice-fish, etc.). The subject into which the lipid nanoparticles are introduced is preferably a human or other mammalian cell.

The method of administering lipid nanoparticles encapsulating nucleic acid to a subject is not particularly limited as long as the lipid nanoparticles can deliver nucleic acid to spleen cells, and can be appropriately selected from known administration methods (e.g., oral administration, parenteral administration (e.g., intranasal administration, intravenous administration, intramuscular administration, topical administration, transdermal administration, subcutaneous administration, intraperitoneal administration, spray, etc.) and the like). As the administration method, intravenously administration is preferred. The dose of the lipid nanoparticles can be appropriately selected in consideration of the type of subject of administration, administration method, and the like.

The lipid nanoparticles of the present invention can be produced as is or by mixing with a pharmaceutically acceptable carrier into oral preparations (e.g., tablet, capsule, etc.) or parenteral agents (e.g., nasal preparation, injection, inhalant, etc.), preferably parenteral agents (more preferably, nasal preparations).

As the pharmaceutically acceptable carrier, those conventionally used as pharmaceutical materials are used. For example, excipient, lubricant, binder, disintegrant, and the like are used in solid preparations, and solvent, solubilizing agent, suspending agent, isotonicity agent, buffering agent, soothing agent, and the like are used in liquid preparations. Furthermore, formulation additives such as antiseptic, antioxidant, colorant, and sweeteners can also be used as necessary.

For example, in the case of a nasal agent, it is used in the form of nasal drops or spray.

### [Example]

The Examples of the present invention are explained in further detail in the following, but the present invention is not limited in any way by the following Examples.

In the following Examples, the ionic lipid (1) is shown by the names listed in the aforementioned Tables. The abbreviations used in the following Examples mean the following.
Chol: cholesterol
CHEMS: cholesteryl hemisuccinate
POPC: 1-palmitoyl-2-oleoyl-sn-glycero-3-phosphocholine
DOPC: 1,2-dioleoyl-sn-glycero-3-phosphocholine
DSPC: 1,2-distearoyl-sn-glycero-3-phosphocholine
SOPC: 1-stearoyl-2-oleoyl-sn-glycero-3-phosphocholine
DEPC: 1,2-dierucoyl-sn-glycero-3-phosphocholine
POPE: 1-palmitoyl-2-oleoyl-sn-glycero-3-phosphoethanolamine
DOPE: 1,2-dioleoyl-sn-glycero-3-phosphoethanolamine
DOPG: 1,2-dioleoyl-sn-glycero-3-phosphoglycerol
DMPG: 1,2-dimyristoyl-sn-glycero-3-phosphoglycerol
DPPG: 1,2-dipalmitoyl-sn-glycero-3-phosphoglycerol
DSPG: 1,2-distearoyl-sn-glycero-3-phosphoglycerol
POPG: 1-palmitoyl-2-oleoyl-sn-glycero-3-phosphoglycerol
SOPG: 1-stearoyl-2-oleoyl-sn-glycero-3-phosphoglycerol
DLoPS: 1,2-dilinoleoyl-sn-glycero-3-phosphoserine
DMG-PEG2000: 1,2-dimyristoyl-rac-glycerol, methoxypolyethylene glycol (number average molecular weight (Mn) of PEG: 2000)
MES: 2-morpholinoethanesulfonic acid
DiR: 1,1'-dioctadecyl-3,3,3',3'-tetramethylindotricarbocyanine, iodide
PBS: phosphate buffered saline

The mol% of the lipid composition indicates mol% relative to the total of ionic lipid (1), anionic phospholipid or anionic cholesterol (2), neutral phospholipid, and cholesterol.

### [Production Example 1] Preparation of mRNA-encapsulated LNP

### (1) Preparation of lipid alcohol solution

Each lipid was dissolved in ethanol to give 10 mM SS-OP, 5 mM phospholipid, 5 mM CHEMS, 10 mM Chol, and 1 mM DMG-PEG2000. From these solutions, lipids were selected and mixed in any ratio such that the total mol amount was 1000 nmol, and 1 mM DMG-PEG2000 ethanol solution was added thereto to give the desired ratio. Finally, ethanol was added to give a total volume of 450 µL to prepare an ethanol solution of the lipid.

A tert-butanol solution of the lipid was also prepared by a similar method.

### (2) Preparation of acidic buffer solution of nucleic acid

The acidic buffer solution of nucleic acid was prepared by mixing mRNA encoding the luciferase gene (luc-mRNA) with 20 mM acidic malic acid buffer (pH 3.0) containing 30 mM NaCl to a concentration of 0.0067 µg/µL.

### (3) Preparation of LNP by alcohol dilution method

Using a NanoAssmblr (registered trademark) ultrafast nanomedicine production device (manufactured by Precision NanoSystems), 1200 µL of acidic buffer solution of nucleic acid and 300 µL of lipid alcohol solution were mixed at flow rates of 3 mL/min and 1 mL/min, respectively, to collect 1 mL of LNP solution. The obtained LNP was diluted with 3 mL of 20 mM MES buffer (pH 6.5) and transferred to Amicon Ultra 4 (Millipore). The transferred LNP solution was ultrafiltered under centrifugation conditions (25°C, 1000 g, about 10 min) to concentrate same to about 500 µL. The obtained concentrate was diluted to 4 mL with PBS, and concentrated again to about 200 µL by ultrafiltration under centrifugal conditions (25°C, 1000 g, about 10 min). Finally, the volume was increased with PBS to a nucleic acid concentration of 10 µg/mL.

### [Experimental Example 1] Measurement of particle size and surface potential of various mRNA-encapsulated LNPs

The particle size and surface potential of the mRNA-encapsulated LNPs of Examples 1 to 10, having different lipid compositions, prepared in Production Example 1, and the mRNA-encapsulated LNPs of Comparative Examples 1 and 2, were measured by dynamic scattering method (Zetasizer Nano; Malvern). The results are shown in Table 2. All LNPs had a preferred particle size and a surface charge of -3 mV to -15 mV. The LNPs of Examples 11 to 32 and Comparative Examples 3 and 4 were prepared with the compositions shown in Table 3.

**[Table 2]**

| | lipid composition | particle size (nm) | PdI | zeta potential (mV) |
|---|---|---|---|---|
| Example 1 | SS-OP/DOPG/Chol/DMG-PEG2000 =52.5/7.5/40/1.0 | 112 | 0.01 | -11 |
| Example 2 | SS-OP/DOPC/CHEMS/Chol/DMG-PEG2000 =52.5/7.5/7.5/32.5/1.0 | 99 | 0.08 | -8 |
| Example 3 | SS-OP/DLoPS/Chol/DMG-PEG2000 =52.5/7.5/40/1.0 + DiR 0.2% | 107 | 0.05 | -12 |
| Example 4 | SS-OP/DOPG/DOPC/Chol/DMG-PEG2000 =50/2.5/7.5/40/1.0 | 140 | 0.11 | -14 |
| Example 5 | SS-OP/DOPG/DSPC/Chol/DMG-PEG2000 =50/2.5/7.5/40/1.0 | 133 | 0.14 | -15 |
| Example 6 | SS-OP/DOPG/DEPC/Chol/DMG-PEG2000 =50/2.5/7.5/40/1.0 | 140 | 0.13 | -15 |
| Example 7 | SS-OP/DOPG/POPC/Chol/DMG-PEG2000 =50/2.5/7.5/40/1.0 | 143 | 0.15 | -13 |
| Example 8 | SS-OP/DOPG/SOPC/Chol/DMG-PEG2000 =50/2.5/7.5/40/1.0 | 133 | 0.11 | -14 |
| Example 9 | SS-OP/DOPG/DOPE/Chol/DMG-PEG2000 =50/2.5/7.5/40/1.0 | 160 | 0.09 | -12 |
| Example 10 | SS-OP/DOPG/POPE/Chol/DMG-PEG2000 =50/2.5/7.5/40/1.0 | 147 | 0.16 | -13 |
| Comparative Example 1 | SS-OP/DOPC/Chol/DMG-PEG2000 =52.5/7.5/40/1.0 | 112 | 0.09 | -3 |
| Comparative Example 2 | SS-OP/DOPC/Chol/DMG-PEG2000 =52.5/7.5/40/1.0 + DiR 0.2% | 103 | 0.06 | -3 |

| | | | | |
|---|---|---|---|---|
| (in the Table, composition ratio is in mol%, particle size is zeta mean) | | | | |

**[Table 3]**

| | lipid composition |
|---|---|
| Example 11 | SS-OP/DOPG/DEPC/Chol/DMG-PEG2000=60/5/5/30/1.0 |
| Example 12 | SS-OP/DOPG/DOPC/Chol/DMG-PEG2000=50/5/5/40/0.5 |
| Example 13 | SS-OP/DOPG/DSPC/Chol/DMG-PEG2000=45/3.75/11.25/40/1.0 |
| Example 14 | SS-OP/DOPG/DEPC/Chol/DMG-PEG2000=45/11.25/3.75/40/1.5 |
| Example 15 | SS-OP/DOPG/DEPC/Chol/DMG-PEG2000=50/7.5/2.5/40/1.5 |
| Example 16 | SS-OP/DOPG/DSPC/Chol/DMG-PEG2000=40/5/15/40/1.0 |
| Example 17 | SS-OP/DOPG/DEPC/Chol/DMG-PEG2000=30/5/15/50/0.5 |
| Example 18 | SS-OP/DOPG/DEPC/Chol/OMG-PEG2000=55/5/5/35/1.0 |
| Example 19 | SS-OP/DOPG/DEPC/Chol/DMG-PEG2000=50/5/5/40/1.0 |
| Example 20 | SS-OP/DOPG/DEPC/Chol/DMG-PEG2000=40/5/5/50/1.0 |
| Example 21 | SS-OP/DOPG/DEPC/Chol/DMG-PEG2000=60/2.5/2.5/35/1.0 |
| Example 22 | SS-OP/DOPG/DEPC/Chol/DMG-PEG2000=50/7.5/7.5/35/1.0 |
| Example 23 | SS-OP/DOPG/DEPC/Chol/DMG-PEG2000=45/10/10/35/1.0 |
| Example 24 | Lipid1/DOPG/DEPC/Chol/DMP-PEG2000=55/5/5/35/1.0 |
| Example 25 | SS-OP/DMPG/DEPC/Chol/DMG-PEG2000=55/5/5/35/1.0 |
| Example 26 | SS-OP/DPPG/DEPC/Chol/DMG-PEG2000=55/5/5/35/1.0 |
| Example 27 | SS-OP/DSPG/DEPC/Chol/DMG-PEG2000=55/5/5/35/1.0 |
| Example 28 | SS-OP/POPG/DEPC/Chol/DMG-PEG2000=55/5/5/35/1.0 |
| Example 29 | SS-OP/SOPG/DEPC/Chol/DMG-PEG2000=55/5/5/35/1.0 |
| Example 30 | SS-EC/DCPG/DEPC/Chol/DMG-PEG2000=55/5/5/35/1.0 |
| Example 31 | SS-OP/SS-EC/DOPG/DEPC/Chol/DMG-PEG2000=35/20/5/5/35/1.0 |
| Example 32 | SS-OP/DLoPS/Chol/DMG-PEG2000=52.5/7.5/40/1.0 |
| Comparative Example 3 | SS-EC/DOPC/Chol/DMG-PEG2000=52.5/7.5/40/1.5 |
| Comparative Example 4 | SS-EC/DOPE/Chol/DSG-PEG2000=64/8/27.5/0.5 |

| | |
|---|---|
| (in the Table, composition ratio is in mol%) | |

### [Experimental Example 2] Evaluation of gene expression activity in spleen - 1 (evaluation of combination with anionic phospholipid or anionic cholesterol)

### (1) I.V. administration to mice

The luc-mRNA-encapsulated LNP prepared according to the method described in Production Example 1 was diluted with PBS to an mRNA concentration of 5.0 µg/mL. The diluted mRNA-encapsulated LNP was intravenously administered to 6-week-old female BALB/c mice at 200 µL per mouse (mRNA dose of 1.0 µg per mouse).

### (2) Measurement of gene expression activity

Six hours after administration, spleens were collected from the mice and frozen at -80°C in homogenization tubes containing cell disruption beads. The frozen tubes were removed and 800 µL of lysis buffer (77 mM Tris, 2 mM EDTA 2Na, 0.1% Triton X-100-containing MQ pH 7.8) was added. Homogenization was repeated twice at -2°C and 4500 rpm for 30 sec using Micro Smash (TOMY). 500 µL of the supernatant was transferred to another tube and centrifuged (4°C, 13000 rpm, 10 min), and 300 µL of the supernatant was collected. 50 µL of Luciferase Substrate (Promega) prepared according to the manufacturer's protocol was added to 20 µL of lysis buffer, and luminescence was measured using Glomax 20/20 luminometer (Promega), which was used as background. Similarly, 50 µL of Luciferase Substrate was added to 20 µL of each sample, and luminescence was measured. 10 µL of the supernatant was diluted 100-fold with MQ to prepare 1 mL of diluted solution, and protein concentration was measured by the BCA method using 25 µL of the diluted solution. Gene expression activity was calculated as relative luminescence units (RLU) by luminescence/(protein concentration x 0.02). The gene expression activity is shown in Fig. 1 as a relative value to Comparative Example 1. As a result, it was confirmed that the LNPs of Example 1 and Example 2 had higher gene expression activity in the spleen than the LNP of Comparative Example 1, which is a composition for liver transfer. Since the gene expression activity depends on the efficiency of nucleic acid delivery into target cells, this shows that the LNPs can efficiently deliver nucleic acids to the spleen.

### [Experimental Example 3] Evaluation of gene expression activity in spleen - 2 (evaluation of combination with PS)

### (1) Preparation of fluorescence-labeled LNP

A lipid alcohol solution containing a fluorescent dye was prepared by adding an alcohol solution of DiR to the lipid alcohol solution described in Production Example 1 such that the amount was 0.2 mol% relative to the total amount of the lipids. This lipid alcohol solution was used to prepare LNPs according to the procedure described in Production Example 1, thereby obtaining fluorescence-labeled LNPs.

### (2) I.V. administration to mouse

The luc-mRNA-encapsulated LNPs prepared according to the method described in Production Example 1 were diluted with PBS to an mRNA concentration of 5.0 µg/mL. The diluted mRNA-encapsulated LNP was intravenously administered to 6-week-old female BALB/c mice at 200 µL per mouse (mRNA dose of 1.0 µg per mouse).

### (3) Evaluation of LNP accumulation and gene expression activity

At 5 hr 55 min after administration, luciferin diluted with PBS to 15 mg/mL was intraperitoneally administered to the mice at 200 µL per mouse. Five min after administration of luciferin (6 hr after administration of LNP), the mouse was euthanized, and the spleen and liver were removed onto a petri dish and imaged using IVIS (PerkinElmer). Using image quantification, LNP accumulation was measured by the fluorescence intensity of DiR, and gene expression was measured by the luminescence intensity of luciferin. Fig. 2 is a graph showing the fluorescence intensity of each LNP, and shows the accumulation amount of fluorescence-labeled LNP in the spleen. Fig. 3 is a graph showing gene expression activity in the spleen, and shows nucleic acid delivery efficiency. It was confirmed that the LNP of Example 3 has high accumulation and nucleic acid delivery efficiency in the spleen compared to the LNP of Comparative Example 2, which is an LNP with a composition for liver transfer.

### [Experimental Example 4] Evaluation of gene expression activity in spleen - 3 (evaluation of combination with neutral phospholipid)

### (1) I.V. administration to mouse

The luc-mRNA-encapsulated LNP prepared according to the method described in Production Example 1 was intravenously administered to mice under the same conditions as in Experimental Example 1 (mRNA dose of 1.0 µg per mouse).

### (2) Measurement of gene expression activity

Measurements were performed using the method described in Experimental Example 2, and gene expression activity was calculated as a relative value to Comparative Example 1. As a result, it was confirmed that gene expression activity in the spleen was higher in both LNP that used DOPG alone, which is an anionic phospholipid, as the phospholipid, and LNPs that combined DOPG with neutral phospholipid (1,2-diacyl-sn-glycero-3-phosphocholine or 1,2-diacyl-sn-glycero-3-phosphoethanolamine), compared to the LNP of Comparative Example 1, which has a composition for liver transfer (Fig. 4).

### [Experimental Example 5] Evaluation of gene expression activity in spleen - 4 (evaluation with various lipid compositions)

### (1) I.V. administration to mouse

The luc-mRNA-encapsulated LNP prepared according to the method described in Production Example 1 was intravenously administered to mice under the same conditions as in Experimental Example 1 (mRNA dose of 1.0 µg per mouse).

### (2) Measurement of gene expression activity

Measurements were performed using the method described in Experimental Example 2, and gene expression activity was calculated as a relative value to Example 5. Gene expression in the spleen was confirmed for LNPs with any lipid composition. Examples 11, 18, 19 and 21 showed particularly high gene expression activity compared to Example 5 (Fig. 5).

### [Experimental Example 6] Evaluation of gene expression activity in spleen - 4 (evaluation using various PGs)

### (1) I.V. administration to mouse

The luc-mRNA-encapsulated LNP prepared according to the method described in Production Example 1 was intravenously administered to mice under the same conditions as in Experimental Example 1 (mRNA dose of 1.0 µg per mouse).

### (2) Measurement of gene expression activity

Measurements were performed using the method described in Experimental Example 2, and gene expression activity was calculated as a relative value to Example 18. Gene expression in the spleen was confirmed for LNPs using any PG. LNP with lipid composition using DOPG or POPG showed particularly high gene expression activity (Fig. 6).

### [Experimental Example 7] Evaluation of gene expression activity in spleen - 6 (evaluation using SS-EC)

### (1) I.V. administration to mouse

The luc-mRNA-encapsulated LNP prepared according to the method described in Production Example 1 was intravenously administered to mice under the same conditions as in Experimental Example 1 (mRNA dose of 1.0 µg per mouse).

### (2) Measurement of gene expression activity

Measurements were performed using the method described in Experimental Example 2, and gene expression activity was calculated as a relative value to Comparative Example 3. Compared to Comparative Example 3, which is an LNP with a composition for delivery to the liver, the LNP of Example 30 showed high gene expression activity in the spleen (Fig. 7).

### [Experimental Example 8] Evaluation of CTL activity using LNP (1) I.V. administration to mouse

LNPs encapsulating mRNA (hereinafter to be referred to as OVA-mRNA) encoding the ovalbumin gene (hereinafter to be referred to as OVA) prepared according to the method described in Production Example 1 were diluted with PBS to an mRNA concentration of 5.0 µg/mL. The diluted mRNA-encapsulated LNPs were intravenously administered to 6-week-old female C57BL/6J mice at 200 µL per mouse (mRNA dose of 1.0 µg per mouse).

### (2) Evaluation of CTL activity

One week after administration, the CTL assay described below was performed to evaluate OVA-specific cytotoxic T lymphocyte (CTL) activity. The following composition was used as the culture medium for splenocytes: RPMI1640 500 mL, FBS 50 mL, 100 mM sodium pyruvate 5 mL, 1 M HEPES 5 mL, 55 mM 2-mercaptoethanol 500 pL, penicillin/streptomycin 5 mL (hereinafter to be referred to as the medium). Untreated mice were euthanized by cervical dislocation, and the spleens were collected. The spleens were cut in the center, and spleen cells were removed from the cut surface using tweezers and suspended in the medium. The cell suspension medium was collected in a 50 mL tube through a 40 µm cell strainer and centrifuged (4°C, 500 g, 5 min). The supernatant was discarded, and 1 mL of Red Blood Cell Lysis Buffer (SIGMA) was added per animal and the mixture was left standing for 5 min. The cells were diluted 5-fold with culture medium and centrifuged (4°C, 500 g, 5 min). The cells were further suspended in 10 mL of culture medium and centrifuged (4°C, 500 g, 5 min). The cells were suspended in 30 mL of culture medium and the number of cells was counted. Then, the cells were passed through a 40 µm cell strainer and divided equally into two 50 mL tubes and centrifuged (4°C, 500 g, 5 min). The cells were suspended in culture medium to a concentration of 1.0×10⁷ cells/mL. (1) To one of the cell suspensions was added 2 mM OVA epitope (consisting of the amino acid sequence of OVA at positions 257-264) in an amount of 1/400 of the cell suspension, and the mixture was left standing in a culture incubator for 1 hr. The epitope-pulsed group was centrifuged (4°C, 500 g, 5 min). After washing with 10 mL of medium, the cells were washed with 10 mL of PBS, and the number of cells was counted again, then suspended at 3.0×10⁷ cells/mL and stained with CFSE at a final concentration of 5 µM to obtain "target cells". (2) The other cell suspension was incubated without adding epitope, and washed with medium and PBS. The cells were suspended at 3.0×10⁷ cells/mL and stained with CFSE at a final concentration of 0.5 µM to obtain "control cells". Both the target cells and the control cells were washed twice with medium and twice with PBS, and suspended at 5.0×10⁷ cells/mL. 100 µL of the "target cells" and 100 µL of the "control cells" were mixed and administered to immunized mice. The both cells can be distinguished by the intensity of CFSE fluorescence. At 20 hr after administration of the cell mixture, the spleens were collected from the mice and analyzed by flow cytometry. The amount of the "target cells" present was corrected by the amount of "control cells" to quantify the epitope-specific CTL activity.

The evaluation results are shown in Figs. 8 and 9. The LNPs with the compositions of Example 18 and Example 24 exhibited CTL activity about 3 times higher than the LNP with the composition of Comparative Example 1, which is an LNP with a composition for delivery to the liver. Furthermore, the LNP with the composition of Example 30 exhibited higher CTL activity than the LNP with the composition of Comparative Example 3, which is an LNP with a composition for delivery to the liver, and the LNP with the composition of Comparative Example 4, which is an LNP with a composition for delivery to the spleen. CTL activity is an index for evaluating whether acquired immunity can be obtained, and shows that the immunity is acquired more as the the CTL activity becomes higher. Therefore, it was shown that the LNPs of Examples 18, 24, and 30 can enhance the acquired immunity more than the Comparative Examples.

### [Experimental Example 9] Evaluation of CTL activity using LNP - 2

### (1) I.V. administration to mouse

OVA-mRNA-encapsulated LNPs prepared according to the method described in Production Example 1 were diluted with PBS to an mRNA concentration of 1.0 µg/mL. The diluted mRNA-encapsulated LNPs were intravenously administered to 6-week-old female C57BL/6J mice at 200 µL per mouse (mRNA dose of 0.2 µg per mouse).

### (2) Evaluation of CTL activity

The CTL activity was evaluated by the method described in [Experimental Example 8]. The results are shown in Fig. 10.

LNP with the composition of Example 31 showed higher CTL activity than Comparative Example 4.

### [Experimental Example 10] Evaluation of therapeutic effect on multiple sclerosis in mice

### (1) Creation of model mice

10-week-old female C57BL/6J mice were pre-bred for one week to be acclimated to the breeding environment. According to the manufacturer's protocol of a kit product (Hooke Laboratories) for inducing experimental autoimmune encephalomyelitis (EAE), which is a multiple sclerosis model, a mixed emulsion of an autoantigen peptide of myelin oligodendrocyte glycoprotein (MOG) (consisting of the amino acid sequence at positions 35-55 of MOG) and complete Freund's adjuvant was subcutaneously administered at 100 µL each to the neck and lumbar regions of the mice. The pertussis toxin stock included in the kit product was diluted with PBS to 165 ng/100 µL. The diluted pertussis toxin was administered intraperitoneally at 100 µL per mouse twice, on the day of administration of the mixed emulsion and the following day.

### (2) I.V. administration to mouse

LNPs encapsulating luc-mRNA or LNPs encapsulating mRNA encoding MOG peptide (consisting of the amino acid sequence at positions 27-63 of MOG) (hereinafter to be referred to as MOG-mRNA) prepared according to the method described in Production Example 1 were diluted with PBS to an mRNA concentration of 1.0 µg/200 µL. The diluted mRNA-encapsulated LNPs were intravenously administered to EAE mice three times, on the 7th, 10th, and 13th days after the day of EAE induction, at 200 µL per mouse.

### (3) Evaluation of therapeutic effect on multiple sclerosis

From the 10th day after the induction of EAE, the pathology score and body weight of the mice were measured every day. The pathology score was determined according to the following criteria: 0: no symptoms, 1: tail droops when lifted from the tail base, 2: paralysis of one hind leg/abnormal gait, 3: paralysis of both hind legs, 3.5: score 3 and obvious decrease in locomotion, 4: paralysis of the front legs, 5: moribund/death. In order to avoid bias in the measurement of the scores, administration of LNP and measurement of the scores were performed by different experimenters, and the scores were measured in a blinded manner.

The evaluation results are shown in Fig. 11 and Fig. 12. It was confirmed that the LNP of Example 32 encapsulating MOG-mRNA alleviated EAE symptoms. Furthermore, the LNP of Example 32 encapsulating luc-mRNA also alleviated EAE symptoms, suggesting that the LNP of Example 32 itself contributes to the alleviation of EAE symptoms.

### [Industrial Applicability]

The lipid nanoparticle of the present invention is useful for delivering nucleic acids to spleen cells.

This application is based on patent application No. 2022-051918 filed in Japan, the contents of which are encompassed in full herein.

## Claims

1. A lipid nanoparticle for use in delivering a nucleic acid to a spleen tissue, comprising
(A) an ionic lipid represented by the formula (1): 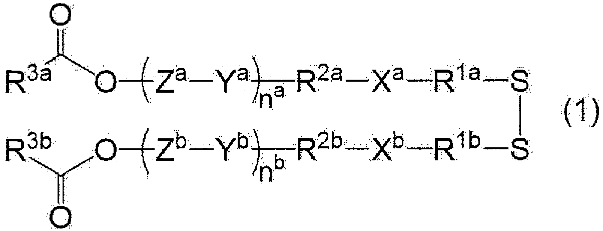 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):
R⁹-O-CO-(CH₂)a- (4)
(in the formula (4),
R⁹ is an aliphatic hydrocarbon group having 2 - 20 carbon atoms, and
a is an integer of 2 - 10)),
(B) an anionic phospholipid or a compound represented by the formula (2): 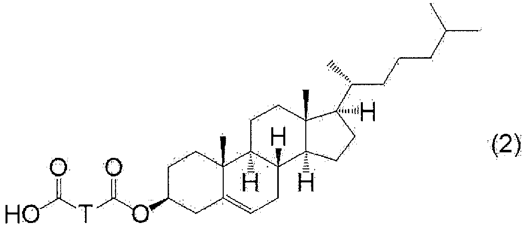 (in the formula (2),
T is a divalent aliphatic hydrocarbon group having 1 - 8 carbon atoms),
(C) cholesterol, and
(D) a dimyristoylglycerol PEG represented by the formula (3):
CH₂(OR⁶)-CH(OR⁷)-CH₂(OR⁸) (3)
(in the formula (3),
any two of R⁶, R⁷ and R⁸ are myristoyl groups, and the remaining one is an alkyl group having 1 - 6 carbon atoms connected via a polyethylene glycol chain with a number average molecular weight of 1,000 to 3,000).

2. The lipid nanoparticle according to claim 1, wherein the anionic phospholipid is 1,2-diacyl-sn-glycero-3-phosphoglycerol or 1,2-diacyl-sn-glycero-3-phosphoserine.

3. The lipid nanoparticle according to claim 1 or 2, wherein the anionic phospholipid is at least one selected from the group consisting of 1,2-dimyristoyl-sn-glycero-3-phosphoglycerol (DMPG), 1,2-dipalmitoyl-sn-glycero-3-phosphoglycerol (DPPG), 1-palmitoyl-2-oleoyl-sn-glycero-3-phosphoglycerol (POPG), 1,2-dioleoyl-sn-glycero-3-phosphoglycerol (DOPG), 1-stearoyl-2-oleoyl-sn-glycero-3-phosphoglycerol (SOPG), 1,2-distearoyl-sn-glycero-3-phosphoglycerol (DSPG), 1,2-dipalmitoyl-sn-glycero-3-phosphoserine (DPPS), 1-palmitoyl-2-oleoyl-sn-glycero-3-phosphoserine (POPS), 1,2-dioleoyl-sn-glycero-3-phosphoserine (DOPS), 1,2-dilinoleoyl-sn-glycero-3-phosphoserine (DLoPS), 1-stearoyl-2-oleoyl-sn-glycero-3-phosphoserine (SOPS), and 1,2-distearoyl-sn-glycero-3-phosphoserine (DSPS).

4. The lipid nanoparticle according to any one of claims 1 to 3, wherein the anionic phospholipid is 1-palmitoyl-2-oleoyl-sn-glycero-3-phosphoglycerol (POPG), 1,2-dioleoyl-sn-glycero-3-phosphoglycerol (DOPG), or 1,2-dilinoleoyl-sn-glycero-3-phosphoserine (DLoPS).

5. The lipid nanoparticle according to any one of claims 1 to 4, wherein T in the formula (2) is a divalent aliphatic hydrocarbon group having 1 - 3 carbon atoms.

6. The lipid nanoparticle according to any one of claims 1 to 5, wherein the ionic lipid represented by the formula (1) is an ionic lipid represented by either the following formula: 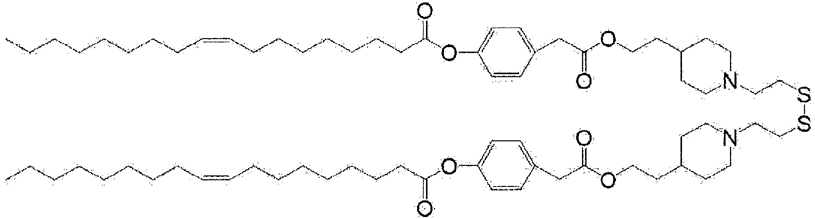 or

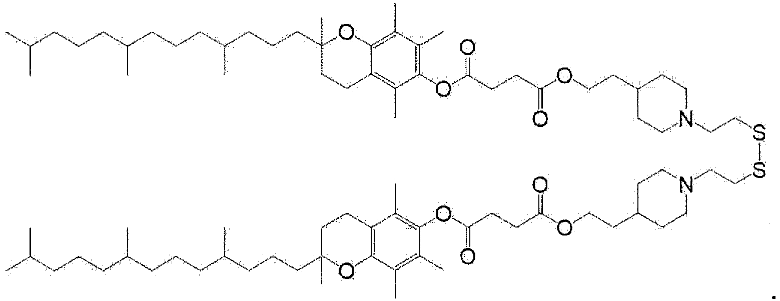

7. The lipid nanoparticle according to any one of claims 1 to 6, wherein a neutral phospholipid is optionally comprised as a component of the lipid nanoparticle.

8. The lipid nanoparticle according to claim 7, wherein the neutral phospholipid is 1,2-diacyl-sn-glycero-3-phosphocholine or 1,2-diacyl-sn-glycero-3-phosphoethanolamine.

9. The lipid nanoparticle according to claim 7 or 8, wherein the neutral phospholipid is at least one selected from the group consisting of 1,2-dioleoyl-sn-glycero-3-phosphocholine (DOPC), 1,2-distearoyl-sn-glycero-3-phosphocholine (DSPC), 1,2-dierucoyl-sn-glycero-3-phosphocholine (DEPC), 1,2-dipalmitoyl-sn-glycero-3-phosphocholine (DPPC), 1-palmitoyl-2-oleoyl-sn-glycero-3-phosphocholine (POPC), 1-stearoyl-2-oleoyl-sn-glycero-3-phosphocholine (SOPC), 1,2-dioleoyl-sn-glycero-3-phosphoethanolamine (DOPE), and 1-palmitoyl-2-oleoyl-sn-glycero-3-phosphoethanolamine (POPE).

10. The lipid nanoparticle according to any one of claims 7 to 9, wherein a ratio of the anionic phospholipid or the compound represented by the formula (2), and the neutral phospholipid is 100:0 to 25:75 mol%.

11. The lipid nanoparticle according to any one of claims 7 to 10, wherein the ionic lipid is 30 to 70 mol%, the anionic phospholipid or the compound represented by the formula (2) is 2.5 to 15 mol%, the neutral phospholipid is 0 to 15 mol%, the cholesterol is 20 to 60 mol%, and the dimyristoylglycerol PEG is 0.5 to 1.5 mol%, relative to the total of the ionic lipid, the anionic phospholipid or the compound represented by the formula (2), the neutral phospholipid, and the cholesterol.

12. A method for delivering a nucleic acid to a spleen tissue, comprising intravenously administering the lipid nanoparticle according to any one of claims 1 to 11 encapsulating the nucleic acid to a living organism.

13. Use of a lipid nanoparticle comprising
(A) an ionic lipid represented by the formula (1): 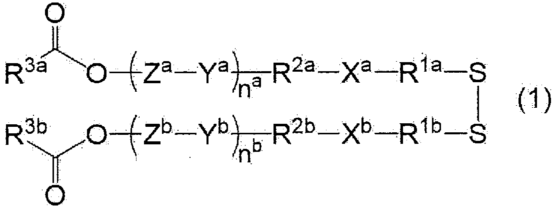 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):
R⁹-O-CO-(CH₂)a- (4)
(in the formula (4),
R⁹ is an aliphatic hydrocarbon group having 2 - 20 carbon atoms, and
a is an integer of 2 - 10)),
(B) an anionic phospholipid or a compound represented by the formula (2): 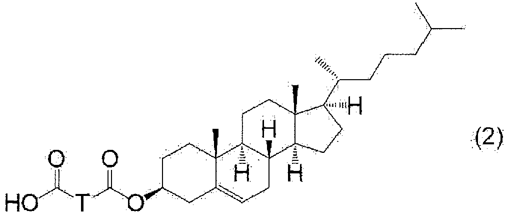 (in the formula (2),
T is a divalent aliphatic hydrocarbon group having 1 - 8 carbon atoms),
(C) cholesterol, and
(D) a dimyristoylglycerol PEG represented by the formula (3):
CH₂(OR⁵)-CH(OR⁷)-CH₂(OR⁸) (3)
(in the formula (3),
any two of R⁶, R⁷ and R⁸ are myristoyl groups, and the remaining one is an alkyl group having 1 - 6 carbon atoms connected via a polyethylene glycol chain with a number average molecular weight of 1,000 to 3,000)
in the production of a medicament for delivering a nucleic acid to a spleen tissue.
